# EUROPEAN PATENT SPECIFICATION

(11) **EP 2 192 174 B1**
(45) Date of publication and mention of the grant of the patent: **11.11.2015**
(21) Application number: 08400048.8
(22) Date of filing: 21.11.2008
(51) Int. Cl.: C12N 5/074

(54) **Reprogramming cells toward a pluripotent state**
Umprogrammierung von Zellen zu einem pluripotenten Status
Reprogrammation de cellules vers un état pluripotent

(43) Date of publication of application: 02.06.2010
(60) Divisional application: 14192115.5
(73) Proprietor: Fraunhofer-Gesellschaft zur Förderung der angewandten Forschung e.V., 80686 München (DE)
(72) Inventor: Stolzing, Alexandra, 04103 Leipzig (DE); Arnold, Antje, 04107 Leipzig (DE); Brown, Jeremy, Falmouth Cornwall TR11 3EW (GB)
(74) Representative: Kilger, Ute

(56) References cited:
- WO-A-2008/087442
- WO-A-2008/097926
- WO-A2-2009/077134
- JAENISCH RUDOLF ET AL: "Stem cells, the molecular circuitry of pluripotency and nuclear reprogramming" CELL, vol. 132, no. 4, February 2008 (2008-02), pages 567-582, XP009114628 ISSN: 0092-8674
- TAKAHASHI K ET AL: "Induction of pluripotent stem cells from mouse embryonic and adult fibroblast cultures by defined factors" CELL, CELL PRESS, CAMBRIDGE, NA, US, vol. 126, no. 4, 25 August 2006 (2006-08-25), pages 663-676, XP003013968 ISSN: 0092-8674
- YU JUNYING ET AL: "Induced pluripotent stem cell lines derived from human somatic cells" SCIENCE, AMERICAN ASSOCIATION FOR THE ADVANCEMENT OF SCIENCE, US, vol. 318, no. 5858, 21 December 2007 (2007-12-21), pages 1917-1920, XP009105055 ISSN: 1095-9203
- DEJOSEZ MARION ET AL: "Ronin is essential for embryogenesis and the pluripotency of mouse embryonic stem cells (vol 133, pg 1162, 2008)" CELL, vol. 134, no. 4, August 2008 (2008-08), page 692, XP009114640 ISSN: 0092-8674
- CHUNG YOUNG ET AL: "Human embryonic stem cell lines generated without embryo destruction", CELL STEM CELL, CELL PRESS, US, vol. 2, no. 2, 7 February 2008 (2008-02-07), pages 113-117, XP002604696, ISSN: 1934-5909, DOI: 10.1016/J.STEM.2007.12.013
- ZUK P A ET AL: "Human adipose tissue is a source of multipotent stem cells", MOLECULAR BIOLOGY OF THE CELL, AMERICAN SOCIETY FOR CELL BIOLOGY, US, vol. 13, no. 12, 20 December 2002 (2002-12-20), pages 4279-4295, XP002249630, ISSN: 1059-1524, DOI: 10.1091/MBC.E02-02-0105
- ANTJE ARNOLD ET AL: "Reprogramming of Human Huntington Fibroblasts Using mRNA", ISRN CELL BIOLOGY,, vol. 2012, 1 January 2012 (2012-01-01), page ArticleID124878, XP009164819,
- Juliane M. Wiehe ET AL: "mRNA-Mediated Gene Delivery Into Human Progenitor Cells Promotes Highly Efficient Protein Expression", Journal of Cellular and Molecular Medicine, vol. 11, no. 3, 1 May 2007 (2007-05-01), pages 521-530, XP55038624, ISSN: 1582-1838, DOI: 10.1111/j.1582-4934.2007.00038.x

### SEQUENCE LISTING

<110> Fraunhofer-Gesellachaft zur Förderung der angewandten Forschung e. V.
<120> Reprogramroing cells toward a pluripotent state
<130> 201763 EP
<160> 28
<170> PatentIn version 3.5
<210> 1
   <211> 2098
   <212> DNA
   <213> Homo sapiens
<400> 1
   attataaatc tagagactcc aggattttaa cgttctgctg gactgagctg gttgcctcat      60
   gttattatgc aggcaactca ctttatccca atttcttgat acttttcctt ctggaggtcc     120
   tatttctcta acatcttcca gaaaagtctt aaagctgcct taaccttttt tccagtccac     180
   ctcttaaatt ttttcctcct cttcctctat actaacatga gtgtggatcc agcttgtccc     240
   caaagcttgc cttgctttga agcatccgac tgtaaagaat cttcacctat gcctgtgatt     300
   tgtgggcctg aagaaaacta tccatccttg caaatgtctt ctgctgagat gcctcacacg     360
   gagactgtct ctcctcttcc ttcctccatg gatctgctta ttcaggacag ccctgattct     420
   tccaccagtc ccaaaggcaa acaacccact tctgcagaga agagtgtcgc aaaaaaggaa     480
   gacaaggtcc cggtcaagaa acagaagacc agaactgtgt tctcttccac ccagctgtgt     540
   gtactcaatg atagatttca gagacagaaa tacctcagcc tccagcagat gcaagaactc     600
   tccaacatcc tgaacctcag ctacaaacag gtgaagacct ggttccagaa ccagagaatg     660
   aaatctaaga ggtggcagaa aaacaactgg ccgaagaata gcaatggtgt gacgcagaag     720
   gcctcagcac ctacctaccc cagcctttac tcttcctacc accagggatg cctggtgaac     780
   ccgactggga accttccaat gtggagcaac cagacctgga acaattcaac ctggagcaac     840
   cagacccaga acatccagtc ctggagcaac cactcctgga acactcagac ctggtgcacc     900
   caatcctgga acaatcaggc ctggaacagt cccttctata actgtggaga ggaatctctg     960
   cagtcctgca tgcagttcca gccaaattct cctgccagtg acttggaggc tgccttggaa    1020
   gctgctgggg aaggccttaa tgtaatacag cagaccacta ggtattttag tactccacaa    1080
   accatggatt tattcctaaa ctactccatg aacatgcaac ctgaagacgt gtgaagatga    1140
   gtgaaactga tattactcaa tttcagtctg gacactggct gaatccttcc tctcccctcc    1200
   tcccatccct cataggattt ttcttgtttg gaaaccacgt gttctggttt ccatgatgcc    1260
   catccagtca atctcatgga gggtggagta tggttggagc ctaatcagcg aggtttcttt    1320
   tttttttttt ttcctattgg atcttcctgg agaaaatact tttttttttt ttttttttga    1380
   aacggagtct tgctctgtcg cccaggctgg agtgcagtgg cgcggtcttg gctcactgca    1440
   agctccgtct cccgggttca cgccattctc ctgcctcagc ctcccgagca gctgggacta    1500
   caggcgcccg ccacctcgcc cggctaatat tttgtatttt tagtagagac ggggtttcac    1560
   tgtgttagcc aggatggtct cgatctcctg accttgtgat ccacccgcct cggcctccct    1620
   aacagctggg atttacaggc gtgagccacc gcgccctgcc tagaaaagac attttaataa    1680
   ccttggctgc cgtctctggc tatagataag tagatctaat actagtttgg atatctttag    1740
   ggtttagaat ctaacctcaa gaataagaaa tacaagtaca aattggtgat gaagatgtat    1800
   tcgtattgtt tgggattggg aggctttgct tattttttaa aaactattga ggtaaagggt    1860
   taagctgtaa catacttaat tgatttctta ccgtttttgg ctctgttttg ctatatcccc    1920
   taatttgttg gttgtgctaa tctttgtaga aagaggtctc gtatttgctg catcgtaatg    1980
   acatgagtac tgctttagtt ggtttaagtt caaatgaatg aaacaactat ttttccttta    2040
   gttgatttta ccctgatttc accgagtgtt tcaatgagta aatatacagc ttaaacat       2098
<210> 2
   <211> 1356
   <212> DNA
   <213> Mus musculus
<400> 2
   tctatcgcct tgagccgttg gccttcagat aggctgattt ggttggtgtc ttgctctttc      60
   tgtgggaagg ctgcggctca cttccttctg acttcttgat aattttgcat tagacattta     120
   actcttcttt ctatgatctt tccttctaga cactgagttt tttggttgtt gcctaaaacc     180
   ttttcagaaa tcccttccct cgccatcaca ctgacatgag tgtgggtctt cctggtcccc     240
   acagtttgcc tagttctgag gaagcatcga attctgggaa cgcctcatca atgcctgcag     300
   tttttcatcc cgagaactat tcttgcttac aagggtctgc tactgagatg ctctgcacag     360
   aggctgcctc tcctcgccct tcctctgaag acctgcctct tcaaggcagc cctgattctt     420
   ctaccagtcc caaacaaaag ctctcaagtc ctgaggctga caagggccct gaggaggagg     480
   agaacaaggt ccttgccagg aagcagaaga tgcggactgt gttctctcag gcccagctgt     540
   gtgcactcaa ggacaggttt cagaagcaga agtacctcag cctccagcag atgcaagaac     600
   tctcctccat tctgaacctg agctataagc aggttaagac ctggtttcaa aaccaaagga     660
   tgaagtgcaa gcggtggcag aaaaaccagt ggttgaagac tagcaatggt ctgattcaga     720
   agggctcagc accagtggag tatcccagca tccattgcag ctatccccag ggctatctgg     780
   tgaacgcatc tggaagcctt tccatgtggg gcagccagac ttggaccaac ccaacttgga     840
   gcagccagac ctggaccaac ccaacttgga acaaccagac ctggaccaac ccaacttgga     900
   gcagccaggc ctggaccgct cagtcctgga acggccagcc ttggaatgct gctccgctcc     960
   ataacttcgg ggaggacttt ctgcagcctt acgtacagtt gcagcaaaac ttctctgcca    1020
   gtgatttgga ggtgaatttg gaagccacta gggaaagcca tgcgcatttt agcaccccac    1080
   aagccttgga attattcctg aactactctg tgactccacc aggtgaaata tgagacttac    1140
   gcaacatctg ggcttaaagt cagggcaaag ccaggttcct tccttcttcc aaatattttc    1200
   atattttttt taaagattta tttattcatt atatgtaagt acactgtagc tgtcttcaga    1260
   cactccagaa gagggcgtca gatcttgtta cgtatggttg tgagccacca tgtggttgct    1320
   gggatttgaa ctcctgacct tcggaagagc agtcgg                              1356
<210> 3
   <211> 2358
   <212> DNA
   <217> Rattus norvegicus
<400> 3
   tcagataggc tgatttcgag tctttctctt ttgtgggaag accgaggctc gcttcttttt      60
   ggcttgttga ctcttttaca tctggacatt taactcttac ttttaagatc tttccctcta     120
   gacactgagt tttaaagtct taactttttg gttgttaaaa actttttttt ttttaaagtc     180
   ccttcccttg ccgttgggct gacatgagcg tggatctttc tggtccccac agtctgccta     240
   gttgtgagga agcatcgaac tctggggatt cctcgccgat gcctgccgtt catcttcctg     300
   aggaaaatta ttcttgctta caagtgtctg ctactgagat gctctgcaca gagactgcct     360
   ctcctccgcc ttcctctggg gacctacctc ttcaagatag ccctgattct tctagcaatc     420
   ccaagctaaa gctgtctggt cccgaggctg acgagggccc tgagaagaaa gaagagaaca     480
   aggtcctcac caagaagcag aagatgcgga ctgtgttctc tcaggcccag ttgtgtgcac     540
   tcaaggatag gtttcagagg caaaggtacc tcagcctcca gcagatgcaa gatctctcta     600
   ccattctgaa cctgagctat aagcaggtga agacctggtt ccaaaaccaa agaatgaagt     660
   gcaagaggtg gcagaaaaac caatggttga agactagcaa cggcctgact cagaagggct     720
   cagcgccggt ggagtatccc agcatccatt gcagctattc tcagggctat ctgatgaacg     780
   cgtctggaaa ccttccagta tggggcagtc agacctggac caacccaact tggaacaacc     840
   agacctggac caacccaacc tggagcaacc agacctggac caacccaact tggagcaacc     900
   aggcctggag cactcagtcc tggtgtactc aggcctggaa cagccagact tggaacgctg     960
   ctccgctcca taacttcggg gaggactccc tgcagcctta tgtgccgttg cagcaaaact    1020
   tctccgccag tgatttggag gcgaatttgg aagccactag ggaaagccag gcgcatttta    1080
   gtaccccgca agccttggaa ttgttcctga actactccgt gaattctcca ggcgaaatat    1140
   gaggtttaca caacaactgg gcttaaagtc agggcagggc cagggtcagc tttcttcctt    1200
   cttccaaaga gttttatatt gttcttattt tttttttaat tattattttg tttttgtttt 1260
   ttgtttatca aggtagggtt tctctgtgtg gttctggctg tcctggaatt cactctgtag 1320
   accaggctgg cctcgtactc agagatctgc ctacttttgc ctcctgaagg ctagggctaa 1380
   agattttcta aagattttca tagtttttat ttttttaatt attatctgtt ttcatgtttg 1440
   tgtttttttg ttttgttttt gtttttgttt ttgtttatca agatagggtt tctctgtgtg 1500
   gctctagtag tcccggaaac tggctctgta gaccaggctg tccttgaact cagaaatctg 1560
   cctttgcctc cggactgcgg ggactaaagg cagtatataa ccacctggca cattgttttt 1620
   atttttattc ttttggtgcc agaaagcaaa cctaggactt tgagctgggc acccactcaa 1680
   ccactgagct ctgtttgcga cccccgtgtt ggctgcattt gtctgagctg ggtaacttgt 1740
   ctttttttcc gtgttaacga tgggcttcgg agacagtgca ctatacactc tatcctcccc 1800
   caggtctcac acacccaccc tactccatac caacccaggc ttgtctgtct tttttttttt 1860
   tggagctgag gactgaaccc agggccttgc gctttctagg caagcgctct acctctgagc 1920
   taaatcccca acccttgtct gtctttttag aagcttgggt cttggtgtgc actgtgtatc 1980
   gttttgaggg gtgaggttta aaagtataca aattataaag attcatgcag atatggtggc 2040
   tcctctcaag gacgagacag aaggatcacc agtttgaggc tatctcagat ataaaataag 2100
   ttcaagacca gcctgtacta tgtctaaata gtaagacagc atctcaacaa aataataaaa 2160
   ctaaggtaag gagataaaag taaagtctca acaaaataca agatctcgcc tgttacagtt 2220
   ctttgatttc ctccgtgtct ttgcagttcc gccaagaggc ttctatgtta atatctgtag 2280
   aaagatgttt atatttgact gtaccatgat aaaccagtgc cagctggact agtttaaata 2340
   aaacactaat tttaccca 2358
<210> 4
   <211> 2518
   <212> DNA
   <213> Homo sapiens
   <00> 4
<210> 5
   <211> 2457
   <212> DNA
   <213> Mus musculus
<400> 5
   ctattaactt gttcaaaaaa gtatcaggag ttgtcaaggc agagaagaga gtgtttgcaa      60
   aaagggaaaa gtactttgct gcctctttaa gactagggct gggagaaaga agaggagaga     120
   gaaagaaagg agagaagttt ggagcccgag gcttaagcct ttccaaaaac taatcacaac     180
   aatcgcggcg gcccgaggag gagagcgcct gttttttcat cccaattgca cttcgcccgt      240
   ctcgagctcc gcttcccccc aactattctc cgccagatct ccgcgcaggg ccgtgcacgc     300
   cgaggccccc gcccgcggcc cctgcatccc ggcccccgag cgcggccccc acagtcccgg      360
   ccgggccgag ggttggcggc cgccggcggg ccgcgcccgc ccagcgcccg catgtataac     420
   atgatggaga cggagctgaa gccgccgggc ccgcagcaag cttcgggggg cggcggcgga     480
   ggaggcaacg ccacggcggc ggcgaccggc ggcaaccaga agaacagccc ggaccgcgtc     540
   aagaggccca tgaacgcctt catggtatgg tcccgggggc agcggcgtaa gatggcccag     600
   gagaacccca agatgcacaa ctcggagatc agcaagcgcc tgggcgcgga gtggaaactt     660
   ttgtccgaga ccgagaagcg gccgttcatc gacgaggcca agcggctgcg cgctctgcac     720
   atgaaggagc acccggatta taaataccgg ccgcggcgga aaaccaagac gctcatgaag     780
   aaggataagt acacgcttcc cggaggcttg ctggcccccg gcgggaacag catggcgagc     840
   ggggttgggg tgggcgccgg cctgggtgcg ggcgtgaacc agcgcatgga cagctacgcg     900
   cacatgaacg gctggagcaa cggcagctac agcatgatgc aggagcagct gggctacccg     960
   cagcacccgg gcctcaacgc tcacggcgcg gcacagatgc aaccgatgca ccgctacgac    1020
   gtcagcgccc tgcagtacaa ctccatgacc agctcgcaga cctacatgaa cggctcgccc    1080
   acctacagca tgtcctactc gcagcagggc acccccggta tggcgctggg ctccatgggc    1140
   tctgtggtca agtccgaggc cagctccagc ccccccgtgg ttacctcttc ctcccactcc    1200
   agggcgccct gccaggccgg ggacctccgg gacatgatca gcatgtacct ccccggcgcc    1260
   gaggtgccgg agcccgctgc gcccagtaga ctgcacatgg cccagcacta ccagagcggc    1320
   ccggtgcccg gcacggccat taacggcaca ctgcccctgt cgcacatgtg agggctggac    1380
   tgcgaactgg agaaggggag agattttcaa agagatacaa gggaattggg aggggtgcaa    1440
   aaagaggaga gtaggaaaaa tctgataatg ctcaaaagga aaaaaaatct ccgcagcgaa    1500
   acgacagctg cggaaaaaaa ccaccaatcc catccaaatt aacgcaaaaa ccgtgatgcc    1560
   gactagaaaa cttttatgag agatcttggg acttcttttt gggggactat ttttgtacag 1620
   agaaaacctg agggcggcgg ggagggcggg ggaatcggac catgtataga tctggaggaa 1680
   aaaaactacg caaaactttt ttttaaagtt ctagtggtac gttaggcgct tcgcagggag 1740
   ttcgcaaaag tctttaccag taatatttag agctagactc cgggcgatga aaaaaaagtt 1800
   ttaatatttg caagcaactt ttgtacagta tttatcgaga taaacatggc aatcaaatgt 1860
   ccattgttta taagctgaga atttgccaat atttttcgag gaaagggttc ttgctgggtt 1920
   ttgattctgc agcttaaatt taggaccgtt acaaacaagg aaggagttta ttcggatttg 1980
   aacattttag ttttaaaatt gtacaaaagg aaaacatgag agcaagtact ggcaagaccg 2040
   ttttcgtggt cttgtttaag gcaaacgttc tagattgtac taaattttta acttactgtt 2100
   aaaggcaaaa aaaaaatgtc catgcaggtt gatatcgttg gtaatttata atagcttttg 2160
   ttcaatccta ccctttcatt ttgttcacat aaaaaatatg gaattactgt gtttgaaata 2220
   ttttcttatg gtttgtaata tttctgtaaa ttgtgatatt ttaaggtttt tccccccttt 2280
   tattttccgt agttgtattt taaaagattc ggctctgtta ttggaatcag gctgccgaga 2340
   atccatgtat atatttgaac taataccatc cttataacag ctacattttc aacttaagtt 2400
   tttactccat tatgcacagt ttgagataaa taaatttttg aaatatggac actgaaa 2457
<210> 6
   <211> 2323
   <212> DNA
   <213> Rattus norvegicus
<400> 6
   gtgtttgcaa aaagggaaaa gtactttgct gcctctttaa gactagggct gggagaaaga 60
   agaggagaga aaaagaaagg agagaagttt ggagcccgag gcttaagcct ttccaaaaac 120
   taatcacaac aatcgcggcg gcccgaggag gagagcgact gttttttcat cccaattgca 180
   cttcgcccgt ctcgagctcc gcttcccccc aactattctc cgccagatct ccgcgcaagg 240
   ccgtgcacgc cgacgacccc gcccgcggcc cctgcatccc ggcccccgcg cgcggccccc 300
   gcagtcccgg ccgggccgag ggtcggcggc cgccggcggg ccgcgcccgc gcccagcgcc 360
   cgcatgtata acatgatgga gacggagctg aagccgccgg gccctcagca agcttcgggg 420
   ggcggcggcg gaggaggcaa cgccacggcg gcggcgaccg gcggcaacca gaagaacagc 480
   ccggaccgcg tcaagaggcc catgaatgcc ttcatggtgt ggtcccgggg gcagcggcgt 540
   aagatggccc aggagaaccc caagatgcac aactcggaga tcagcaagcg cctgggcgcc 600
   gagtggaaac ttttgtcgga gaccgagaag cggccgttca tcgacgaggc caagcggctg 660
   cgcgctctgc acatgaagga gcacccggat tataaatacc ggccgcggcg gaaaaccaag 720
   acgctcatga agaaggataa gtacacgctt cccggaggct tgctggcccc cggcgggaac 780
   agcatggcga gcggggttgg ggtgggcgcc ggcctgggtg cgggcgtgaa ccagcgcatg    840
   gacagctacg cgcacatgaa cggctggagc aacggcagct acagcatgat gcaggagcag    900
   ctgggctacc cgcagcaccc gggcctcaac gctcacggcg cggcacagat gcagccgatg    960
   caccgctacg acgtcagcgc cctgcagtac aactccatga ccagctcgca gacctacatg   1020
   aacggctcgc ccacctacag catgtcctac tcgcagcagg gcacccccgg tatggcgctg   1080
   ggctccatgg gctctgtggt caagtccgag gccagttcca gcccccccgt ggttacctct   1140
   tcctcccact ccagggcgcc ctgccaggcc ggggacctcc gggacatgat cagcatgtac   1200
   ctccccggcg ccgaggtgcc ggagcccgct gcgcccagta gactgcacat ggcccagcac   1260
   taccagagcg gcccggtgcc cggcacggcc attaacggca cactgcccct gtcgcacatg   1320
   tgagggccgg accgcgaact ggagaagggg agagattttt caaaaagata caagggaatt   1380
   gggaggggtg caaaagagga gagtaagaaa aatctgaatg ctcaaaagga aaaaaaaaat   1440
   ctcattaccc gcagcaaaat gacagctgcg gaaaaaaacc accaatccca tccaaattaa   1500
   cgcaaaaacc gtgatgccga ctagaaaact tttatgagag atctggagga aaaaaactac   1560
   gcaaaacttt tttttaaagt tctagtggta cgttaggcgc ttcgcaggga gttctcaaaa   1620
   gtctttacca gtaatattta gaactagact ccgggcgatg aaaaaagttt taatatttgc   1680
   aagcaacttt tgtacagtat ttatcgagat aaacatggca atcaaatgtc cattgtttat   1740
   aagctgagaa tttgccaata tttttcgagg aaagggttct tgctgggttt tgattctgca   1800
   gcttaaatta aggaccgtta cagacaagga aggaatttat tcggatttga acgttttagt   1860
   tttaaaattg tacaaaagga aaacatgaga gcaagtactg gcaagaccat tttcgtggtc   1920
   ttgtttaggg caaacgttct agattgtact aaatttttaa cttactgtta aaggcaaaaa   1980
   aaaaatgtcc atgcaggttg atatcgttgg taatttataa tagcttttgt tcaatcccac   2040
   ccttttcatt ttgttcacat aaaaatatgg aaattactgt gtttgaaata ttttcttatg   2100
   gtttgtaata tttctgtaaa ttgtgatatt ttaaggtttt ttcccccttt tattttccgt   2160
   agttgtattt taaaagattc ggctgttatt ggaaccaggc tgccgagaat ccatgtatat   2220
   atttgaacta ataccatcct tataacagtt acgtttccaa cttaagtttt tactccatta   2280
   tgcacagttt gagataaata aatttttgaa atatggacac tga                     2323
<210> 7
   <211> 1411
   <212> DNA
   <213> Homo sapiens
<400> 7
   ccttcgcaag ccctcatttc accaggcccc cggcttgggg cgccttcctt ccccatggcg     60
   ggacacctgg cttcggattt cgccttctcg ccccctccag gtggtggagg tgatgggcca    120
   ggggggccgg agccgggctg ggttgatcct cggacctggc taagcttcca aggccctcct    180
   ggagggccag gaatcgggcc gggggttggg ccaggctctg aggtgtgggg gattccccca   240
   tgccccccgc cgtatgagtt ctgtgggggg atggcgtact gtgggcccca ggttggagtg   300
   gggctagtgc cccaaggcgg cttggagacc tctcagcctg agggcgaagc aggagtcggg   360
   gtggagagca actccgatgg ggcctccccg gagccctgca ccgtcacccc tggtgccgtg   420
   aagctggaga aggagaagct ggagcaaaac ccggaggagt cccaggacat caaagctctg   480
   cagaaagaac tcgagcaatt tgccaagctc ctgaagcaga agaggatcac cctgggatat   540
   acacaggccg atgtggggct caccctgggg gttctatttg ggaaggtatt cagccaaacg   600
   accatctgcc gctttgaggc tctgcagctt agcttcaaga acatgtgtaa gctgcggccc   660
   ttgctgcaga agtgggtgga ggaagctgac aacaatgaaa atcttcagga gatatgcaaa   720
   gcagaaaccc tcgtgcaggc ccgaaagaga aagcgaacca gtatcgagaa ccgagtgaga   780
   ggcaacctgg agaatttgtt cctgcagtgc ccgaaaccca cactgcagca gatcagccac   840
   atcgcccagc agcttgggct cgagaaggat gtggtccgag tgtggttctg taaccggcgc   900
   cagaagggca agcgatcaag cagcgactat gcacaacgag aggattttga ggctgctggg   960
   tctcctttct cagggggacc agtgtccttt cctctggccc cagggcccca ttttggtacc  1020
   ccaggctatg ggagccctca cttcactgca ctgtactcct cggtcccttt ccctgagggg  1080
   gaagcctttc cccctgtctc cgtcaccact ctgggctctc ccatgcattc aaactgaggt  1140
   gcctgccctt ctaggaatgg gggacagggg gaggggagga gctagggaaa gaaaacctgg  1200
   agtttgtgcc agggtttttg ggattaagtt cttcattcac taaggaagga attgggaaca  1260
   caaagggtgg gggcagggga gtttggggca actggttgga gggaaggtga agttcaatga  1320
   tgctcttgat tttaatccca catcatgtat cacttttttc ttaaataaag aagcctggga    1380
   cacagtagat agacacactt aaaaaaaaaa a                                 1411
<210> 8
   <211> 1346
   <212> DNA
   <213> Mus musculus
<400>
   aaccgtccct aggtgagccg tctttccacc aggcccccgg ctcggggtgc ccaccttccc    60
   catggctgga cacctggctt cagacttcgc cttctcaccc ccaccaggtg ggggtgatgg    120
   gtcagcaggg ctggagccgg gctgggtgga tcctcgaacc tggctaagct tccaagggcc   180
   tccaggtggg cctggaatcg gaccaggctc agaggtattg gggatctccc catgtccgcc   240
   cgcatacgag ttctgcggag ggatggcata ctgtggacct caggttggac tgggcctagt   300
   cccccaagtt ggcgtggaga ctttgcagcc tgagggccag gcaggagcac gagtggaaag   360
   caactcagag ggaacctcct ctgagccctg tgccgaccgc cccaatgccg tgaagttgga     420
   gaaggtggaa ccaactcccg aggagtccca ggacatgaaa gccctgcaga aggagctaga     480
   acagtttgcc aagctgctga agcagaagag gatcaccttg gggtacaccc aggccgacgt      540
   ggggctcacc ctgggcgttc tctttggaaa ggtgttcagc cagaccacca tctgtcgctt     600
   cgaggccttg cagctcagcc ttaagaacat gtgtaagctg cggcccctgc tggagaagtg     660
   ggtggaggaa gccgacaaca atgagaacct tcaggagata tgcaaatcgg agaccctggt     720
   gcaggcccgg aagagaaagc gaactagcat tgagaaccgt gtgaggtgga gtctggagac     780
   catgtttctg aagtgcccga agccctccct acagcagatc actcacatcg ccaatcagct     840
   tgggctagag aaggatgtgg ttcgagtatg gttctgtaac cggcgccaga agggcaaaag     900
   atcaagtatt gagtattccc aacgagaaga gtatgaggct acagggacac ctttcccagg     960
   gggggctgta tcctttcctc tgcccccagg tccccacttt ggcaccccag gctatggaag    1020
   cccccacttc accacactct actcagtccc ttttcctgag ggcgaggcct ttccctctgt    1080
   tcccgtcact gctctgggct ctcccatgca ttcaaactga ggcaccagcc ctccctgggg    1140
   atgctgtgag ccaaggcaag ggaggtagac aagagaacct ggagctttgg ggttaaattc    1200
   ttttactgag gagggattaa aagcacaaca ggggtggggg gtgggatggg gaaagaagct    1260
   cagtgatgct gttgatcagg agcctggcct gtctgtcact catcattttg ttcttaaata    1320
   aagactggga cacacagtag atagct                                         1346
<210> 9
   <211> 2949
   <212> DNA
   <213> Homo sapiens
<400> 9
<210> 10
   <211> 3057
   <212> DNA
   <213> Mus musculus
<400> 10
   agttccccgg ccaagagagc gagcgcggct ccgggcgcgc ggggagcaga ggcggtggcg 60
   ggcggcggcg gcacccggag ccgccgagtg cccctccccg cccctccagc cccccaccca 120
   gcaacccgcc cgtgacccgc gcccatggcc gcgcgcaccc ggcacagtcc ccaggactcc 180
   gcaccccgcg ccaccgccca gctcgcagtt ccgcgccacc gcggccattc tcacctggcg 240
   gcgccgcccg cccaccgccc ggaccacagc ccccgcgccg ccgacagcca cagtggccgc 300
   gacaacggtg ggggacactg ctgagtccaa gagcgtgcag cctggccatc ggacctactt 360
   atctgccttg ctgattgtct atttttataa gagtttacaa cttttctaag aatttttgta 420
   tacaaaggaa cttttttaaa gacatcgccg gtttatattg aatccaaaga agaaggatct 480
   cgggcaatct gggggttttg gtttgaggtt ttgtttctaa agtttttaat cttcgttgac 540
   tttggggctc aggtacccct ctctcttctt cggactccgg aggaccttct gggcccccac 600
   attaatgagg cagccacctg gcgagtctga catggctgtc agcgacgctc tgctcccgtc 660
   cttctccacg ttcgcgtccg gcccggcggg aagggagaag acactgcgtc cagcaggtgc 720
   cccgactaac cgttggcgtg aggaactctc tcacatgaag cgacttcccc cacttcccgg 780
   ccgcccctac gacctggcgg cgacggtggc cacagacctg gagagtggcg gagctggtgc 840
   agcttgcagc agtaacaacc cggccctcct agcccggagg gagaccgagg agttcaacga 900
   cctcctggac ctagacttta tcctttccaa ctcgctaacc caccaggaat cggtggccgc 960
   caccgtgacc acctcggcgt cagcttcatc ctcgtcttcc ccggcgagca gcggccctgc 1020
   cagcgcgccc tccacctgca gcttcagcta tccgatccgg gccgggggtg acccgggcgt 1080
   ggctgccagc aacacaggtg gagggctcct ctacagccga gaatctgcgc cacctcccac 1140
   ggcccccttc aacctggcgg acatcaatga cgtgagcccc tcgggcggct tcgtggctga 1200
   gctcctgcgg ccggagttgg acccagtata cattccgcca cagcagcctc agccgccagg 1260
   tggcgggctg atgggcaagt ttgtgctgaa ggcgtctctg accacccctg gcagcgagta 1320
   cagcagccct tcggtcatca gtgttagcaa aggaagccca gacggcagcc accccgtggt 1380
   agtggcgccc tacagcggtg gcccgccgcg catgtgcccc aagattaagc aagaggcggt 1440
   cccgtcctgc acggtcagcc ggtccctaga ggcccatttg agcgctggac cccagctcag 1500
   caacggccac cggcccaaca cacacgactt ccccctgggg cggcagctcc ccaccaggac 1560
   tacccctaca ctgagtcccg aggaactgct gaacagcagg gactgtcacc ctggcctgcc 1620
   tcttccccca ggattccatc cccatccggg gcccaactac cctcctttcc tgccagacca 1680
   gatgcagtca caagtcccct ctctccatta tcaagagctc atgccaccgg gttcctgcct 1740
   gccagaggag cccaagccaa agaggggaag aaggtcgtgg ccccggaaaa gaacagccac 1800
   ccacacttgt gactatgcag gctgtggcaa aacctatacc aagagttctc atctcaaggc 1860
   acacctgcga actcacacag gcgagaaacc ttaccactgt gactgggacg gctgtgggtg 1920
   gaaattcgcc cgctccgatg aactgaccag gcactaccgc aaacacacag ggcaccggcc 1980
   ctttcagtgc cagaagtgtg acagggcctt ttccaggtcg gaccaccttg ccttacacat 2040
   gaagaggcac ttttaaatcc cacgtagtgg atgtgaccca cactgccagg agagagagtt 2100
   cagtattttt ttttctaacc tttcacactg tcttcccacg aggggaggag cccagctggc 2160
   aagcgctaca atcatggtca agttcccagc aagtcagctt gtgaatggat aatcaggaga 2220
   aaggaagagt tcaagagaca aaacagaaat actaaaaaca aacaaacaaa aaaacaaaca 2280
   aaaaaaacaa gaaaaaaaaa tcacagaaca gatggggtct gatactggat ggatcttcta 2340
   tcattccaat accaaatcca acttgaacat gcccggactt acaaaatgcc aaggggtgac 2400
   tggaagtttg tggatatcag ggtatacact aaatcagtga gcttgggggg agggaagacc 2460
   aggattccct tgaattgtgt ttcgatgatg caatacacac gtaaagatca ccttgtatgc 2520
   tctttgcctt cttaaaaaaa aaaaaagcca ttattgtgtc ggaggaagag gaagcgattc 2580
   aggtacagaa catgttctaa cagcctaaat gatggtgctt ggtgagtcgt ggttctaaag 2640
   gtaccaaacg ggggagccaa agttctccaa ctgctgcata cttttgacaa ggaaaatcta 2700
   gttttgtctt ccgatctaca ttgatgacct aagccaggta aataagcctg gtttatttct 2760
   gtaacatttt tatgcagaca gtctgttatg cactgtggtt tcagatgtgc aataatttgt 2820
   acaatggttt attcccaagt atgcctttaa gcagaacaaa tgtgtttttc tatatagttc 2880
   cttgccttaa taaatatgta atataaattt aagcaaactt ctattttgta tatttgtaaa 2940
   ctacaaagta aaaaaaaatg aacattttgt ggagtttgta ttttgcatac tcaaggtgag 3000
   aaataagttt taaataaacc tataatattt tatctgaacg acaaaaaaaa aaaaaaa 3057
<210> 11
   <211> 2393
   <212> DNA
   <213> Rattus norvegicus
<400> 11
   atcttcgttg acttcggggt ttgggtaccc ctctctcttc ttcggactcc ggaggacctt      60
   ctgggccccc acattaatga ggcagccacc tggcgagtct gacatggctg tcagcgacgc     120
   tctgctcccg tccttctcca cgttcgcgtc cggcccggcg ggaagggaga agacactgcg     180
   tccagcaggt gccccgacta accgttggcg agaggaactc tctcacatga agcgacttcc     240
   cccacttccc ggccgcccct acgacctggc ggcgacggtg gccacagacc tggaaagtgg     300
   tggagctggt gcagcttgca gcagtaacaa cccggcccta ccccggaggg agaccgagga     360
   gttcaacgat ctcctggacc tagactttat cctttccaac tcgctatccc accaggaatc     420
   ggtggccgcc accgtgacca cctcggcgtc agcttcatcc tcgtcttccc cagctagcag     480
   cggccctgcc agcgcgccct ccacctgcag cttcagctat ccgatccggg ccgggggtga     540
   cccgggcgtg gctgcgggca acacaggtgg agggctcctc tacagccgag aatctgcgcc     600
   acctcccacg gcccccttca acctggcgga catcaatgac gtgagcccct cgggcggctt     660
   cgtggctgag ctcctgcggc cggagttgga cccagtatac attccgccac agcagcctca     720
   gccgccaggt ggcgggctga tgggcaagtt tgtgctgaag gcgtctctga gcacccctgg     780
   cagcgagtac accagccctt cggtcatcag tgttagcaaa ggaagcccag acggcagcca     840
   ccctgtggta gtggcgccct acagcggtgg cccgccgcgt atgtgcccca agattaagca     900
   agaggccgtc ccgtcctgca cggtcagccg gtccctagag gcccacttga gcgctggacc     960
   ccagctcagc aacggccaca ggcccaacac acacgacttc cccctggggc ggcagctccc    1020
   caccaggact acccctacac tgagtcccga ggaactgctg aacagcaggg actgtcaccc    1080
   tggcctgcct cttcccccag gattccatcc ccatccgggg cccagctacc ctcctttcct    1140
   gccagaccag atgcagtcgc aagtcccctc tctccattat caagagctca tgccaccggg    1200
   atcctgcctg ccagaggagc ccaagccaaa gaggggaaga aggtcttggc cccggaaaag    1260
   aacagccacc cacacttgtg actatgcagg ctgtggcaaa acctatacga agagttctca    1320
   tctcaaggca cacctgcgaa ctcacacagg cgagaaacct taccactgtg actgggacgg    1380
   ctgtgggtgg aaattcgccc gctcagatga actgaccagg cactaccgca aacacaccgg    1440
   gcaccggccc tttcagtgcc agaagtgcga cagggccttt tccaggtcgg accaccttgc    1500
   cttacacatg aagaggcact tttaaattcc acatcgtgga catgacccac actgccagga    1560
   gagagttcag tatttttttt taacctttca cactgtcttc ccacgagggg aggagcccag    1620
   ctggcaagcg ctacaatcat ggtcaagttc ccagcaagtc agcttgtgaa tggataatca    1680
   ggagaaagga agagtccaag ggacaaaaga aaagaaaaga aaaaaatact aaaaaacaaa    1740
   caaacaaaaa aaaaaaacaa aagaaaaaaa tcacagaaca gatggggtct gagactggat 1800
   cttctatcat tccaatacca aatccgactt gaacaagact ggacttacaa aatgccaagg 1860
   ggtgactgga agtttgtgga tatcagggta tacattaaat cagtgacctg gggggaggga 1920
   agaccagagt tcccttgaat tgtgcttcaa tgatgcaata tacatggaaa gaccaccttg 1980
   tatgctcttt gccttctaaa aagccattat gacgtcagag gaagaggaag caattcaggt 2040
   acagaacgtg ttctaatagc ctaaacgatg gtgcttggtg agtcgtggtt ctaaaggtac 2100
   caaacggggg agccaaagtt ctccaactgc tgcatacttt gacaaggaaa atctattttt 2160
   gtcttccgat ctacatttat gacctaagtc aggtaaataa gcctggttta tttctgtaac 2220
   attttttatg cagacagtct gttatgcact gtggtttcag atgtgcaata atttgtacaa 2280
   tggtttattc ccaagtatgc ctttaagcag aacaaatgtg tttttctata tagttccttg 2340
   ccttaataaa tatgtaatat aaatttaaaa aaaaaaaaaa aaaaaaaaaa aaa 2393
<210> 12
   <211> 4018
   <212> DNA
   <213> Homo sapiens
<400> 12
   caggcagcgc tgcgtcctgc tgcgcacgtg ggaagccctg gccccggcca cccccgcgat 60
   gccgcgcgct ccccgctgcc gagccgtgcg ctccctgctg cgcagccact accgcgaggt 120
   gctgccgctg gccacgttcg tgcggcgcct ggggccccag ggctggcggc tggtgcagcg 180
   cggggacccg gcggctttcc gcgcgctggt ggcccagtgc ctggtgtgcg tgccctggga 240
   cgcacggccg ccccccgccg ccccctcctt ccgccaggtg tcctgcctga aggagctggt 300
   ggcccgagtg ctgcagaggc tgtgcgagcg cggcgcgaag aacgtgctgg ccttcggctt 360
   cgcgctgctg gacggggccc gcgggggccc ccccgaggcc ttcaccacca gcgtgcgcag 420
   ctacctgccc aacacggtga ccgacgcact gcgggggagc ggggcgtggg ggctgctgct 480
   gcgccgcgtg ggcgacgacg tgctggttca cctgctggca cgctgcgcgc tctttgtgct 540
   ggtggctccc agctgcgcct accaggtgtg cgggccgccg ctgtaccagc tcggcgctgc 600
   cactcaggcc cggcccccgc cacacgctag tggaccccga aggcgtctgg gatgcgaacg 660
   ggcctggaac catagcgtca gggaggccgg ggtccccctg ggcctgccag ccccgggtgc 720
   gaggaggcgc gggggcagtg ccagccgaag tctgccgttg cccaagaggc ccaggcgtgg 780
   cgctgcccct gagccggagc ggacgcccgt tgggcagggg tcctgggccc acccgggcag 840
   gacgcgtgga ccgagtgacc gtggtttctg tgtggtgtca cctgccagac ccgccgaaga 900
   agccacctct ttggagggtg cgctctctgg cacgcgccac tcccacccat ccgtgggccg 960
   ccagcaccac gcgggccccc catccacatc gcggccacca cgtccctggg acacgccttg 1020
   tcccccggtg tacgccgaga ccaagcactt cctctactcc tcaggcgaca aggagcagct 1080
   gcggccctcc ttcctactca gctctctgag gcccagcctg actggcgctc ggaggctcgt 1140
   ggagaccatc tttctgggtt ccaggccctg gatgccaggg actccccgca ggttgccccg 1200
   cctgccccag cgctactggc aaatgcggcc cctgtttctg gagctgcttg ggaaccacgc 1260
   gcagtgcccc tacggggtgc tcctcaagac gcactgcccg ctgcgagctg cggtcacccc 1320
   agcagccggt gtctgtgccc gggagaagcc ccagggctct gtggcggccc ccgaggagga 1380
   ggacacagac ccccgtcgcc tggtgcagct gctccgccag cacagcagcc cctggcaggt 1440
   gtacggcttc gtgcgggcct gcctgcgccg gctggtgccc ccaggcctct ggggctccag 1500
   gcacaacgaa cgccgcttcc tcaggaacac caagaagttc atctccctgg ggaagcatgc 1560
   caagctctcg ctgcaggagc tgacgtggaa gatgagcgtg cgggactgcg cttggctgcg 1620
   caggagccca ggggttggct gtgttccggc cgcagagcac cgtctgcgtg aggagatcct 1680
   ggccaagttc ctgcactggc tgatgagtgt gtacgtcgtc gagctgctca ggtctttctt 1740
   ttatgtcacg gagaccacgt ttcaaaagaa caggctcttt ttctaccgga agagtgtctg 1800
   gagcaagttg caaagcattg gaatcagaca gcacttgaag agggtgcagc tgcgggagct 1860
   gtcggaagca gaggtcaggc agcatcggga agccaggccc gccctgctga cgtccagact 1920
   ccgcttcatc cccaagcctg acgggctgcg gccgattgtg aacatggact acgtcgtggg 1980
   agccagaacg ttccgcagag aaaagagggc cgagcgtctc acctcgaggg tgaaggcact 2040
   gttcagcgtg ctcaactacg agcgggcgcg gcgccccggc ctcctgggcg cctctgtgct 2100
   gggcctggac gatatccaca gggcctggcg caccttcgtg ctgcgtgtgc gggcccagga 2160
   cccgccgcct gagctgtact ttgtcaaggt ggatgtgacg ggcgcgtacg acaccatccc 2220
   ccaggacagg ctcacggagg tcatcgccag catcatcaaa ccccagaaca cgtactgcgt 2280
   gcgtcggtat gccgtggtcc agaaggccgc ccatgggcac gtccgcaagg ccttcaagag 2340
   ccacgtctct accttgacag acctccagcc gtacatgcga cagttcgtgg ctcacctgca 2400
   ggagaccagc ccgctgaggg atgccgtcgt catcgagcag agctcctccc tgaatgaggc 2460
   cagcagtggc ctcttcgacg tcttcctacg cttcatgtgc caccacgccg tgcgcatcag 2520
   gggcaagtcc tacgtccagt gccaggggat cccgcagggc tccatcctct ccacgctgct 2580
   ctgcagcctg tgctacggcg acatggagaa caagctgttt gcggggattc ggcgggacgg 2640
   gctgctcctg cgtttggtgg atgatttctt gttggtgaca cctcacctca cccacgcgaa 2700
   aaccttcctc aggaccctgg tccgaggtgt ccctgagtat ggctgcgtgg tgaacttgcg 2760
   gaagacagtg gtgaacttcc ctgtagaaga cgaggccctg ggtggcacgg cttttgttca 2820
   gatgccggcc cacggcctat tcccctggtg cggcctgctg ctggataccc ggaccctgga 2880
   ggtgcagagc gactactcca gctatgcccg gacctccatc agagccagtc tcaccttcaa    2940
   ccgcggcttc aaggctggga ggaacatgcg tcgcaaactc tttggggtct tgcggctgaa    3000
   gtgtcacagc ctgtttctgg atttgcaggt gaacagcctc cagacggtgt gcaccaacat    3060
   ctacaagatc ctcctgctgc aggcgtacag gtttcacgca tgtgtgctgc agctcccatt    3120
   tcatcagcaa gtttggaaga accccacatt tttcctgcgc gtcatctctg acacggcctc    3180
   cctctgctac tccatcctga aagccaagaa cgcagggatg tcgctggggg ccaagggcgc    3240
   cgccggccct ctgccctccg aggccgtgca gtggctgtgc caccaagcat tcctgctcaa    3300
   gctgactcga caccgtgtca cctacgtgcc actcctgggg tcactcagga cagcccagac    3360
   gcagctgagt cggaagctcc cggggacgac gctgactgcc ctggaggccg cagccaaccc    3420
   ggcactgccc tcagacttca agaccatcct ggactgatgg ccacccgccc acagccaggc    3480
   cgagagcaga caccagcagc cctgtcacgc cgggctctac gtcccaggga gggaggggcg   3540
   gcccacaccc aggcccgcac cgctgggagt ctgaggcctg agtgagtgtt tggccgaggc    3600
   ctgcatgtcc ggctgaaggc tgagtgtccg gctgaggcct gagcgagtgt ccagccaagg    3660
   gctgagtgtc cagcacacct gccgtcttca cttccccaca ggctggcgct cggctccacc    3720
   ccagggccag cttttcctca ccaggagccc ggcttccact ccccacatag gaatagtcca    3780
   tccccagatt cgccattgtt cacccctcgc cctgccctcc tttgccttcc acccccacca    3840
   tccaggtgga gaccctgaga aggaccctgg gagctctggg aatttggagt gaccaaaggt    3900
   gtgccctgta cacaggcgag gaccctgcac ctggatgggg gtccctgtgg gtcaaattgg    3960
   ggggaggtgc tgtgggagta aaatactgaa tatatgagtt tttcagtttt gaaaaaaa      4018
<210> 13
   <211> 4237
   <212> DNA
   <213> Mus musculus
<400> 13
   ggttcccgca cgtgggaggc ccatcccggc cttgagcaca atgacccgcg ctcctcgttg      60
   ccccgcggtg cgctctctgc tgcgcagccg ataccgggag gtgtggccgc tggcaacctt     120
   tgtgcggcgc ctggggcccg agggcaggcg gcttgtgcaa cccggggacc cgaagatcta     180
   ccgcactttg gttgcccaat gcctagtgtg catgcactgg ggctcacagc ctccacctgc     240
   cgacctttcc ttccaccagg tgtcatccct gaaagagctg gtggccaggg ttgtgcagag     300
   actctgcgag cgcaacgaga gaaacgtgct ggcttttggc tttgagctgc ttaacgaggc     360
   cagaggcggg cctcccatgg ccttcactag tagcgtgcgt agctacttgc ccaacactgt     420
   tattgagacc ctgcgtgtca gtggtgcatg gatgctactg ttgagccgag tgggcgacga     480
   cctgctggtc tacctgctgg cacactgtgc tctttatctt ctggtgcccc ccagctgtgc     540
   ctaccaggtg tgtgggtctc ccctgtacca aatttgtgcc accacggata tctggccctc 600
   tgtgtccgct agttacaggc ccacccgacc cgtgggcagg aatttcacta accttaggtt 660
   cttacaacag atcaagagca gtagtcgcca ggaagcaccg aaacccctgg ccttgccatc 720
   tcgaggtaca aagaggcatc tgagtctcac cagtacaagt gtgccttcag ctaagaaggc 780
   cagatgctat cctgtcccga gagtggagga gggaccccac aggcaggtgc taccaacccc 840
   atcaggcaaa tcatgggtgc caagtcctgc tcggtccccc gaggtgccta ctgcagagaa 900
   agatttgtct tctaaaggaa aggtgtctga cctgagtctc tctgggtcgg tgtgctgtaa 960
   acacaagccc agctccacat ctctgctgtc accaccccgc caaaatgcct ttcagctcag 1020
   gccatttatt gagaccagac atttccttta ctccagggga gatggccaag agcgtctaaa 1080
   cccctcattc ctactcagca acctccagcc taacttgact ggggccagga gactggtgga 1140
   gatcatcttt ctgggctcaa ggcctaggac atcaggacca ctctgcagga cacaccgtct 1200
   atcgcgtcga tactggcaga tgcggcccct gttccaacag ctgctggtga accatgcaga 1260
   gtgccaatat gtcagactcc tcaggtcaca ttgcaggttt cgaacagcaa accaacaggt 1320
   gacagatgcc ttgaacacca gcccaccgca cctcatggat ttgctccgcc tgcacagcag 1380
   tccctggcag gtatatggtt ttcttcgggc ctgtctctgc aaggtggtgt ctgctagtct 1440
   ctggggtacc aggcacaatg agcgccgctt ctttaagaac ttaaagaagt tcatctcgtt 1500
   ggggaaatac ggcaagctat cactgcagga actgatgtgg aagatgaaag tagaggattg 1560
   ccactggctc cgcagcagcc cggggaagga ccgtgtcccc gctgcagagc accgtctgag 1620
   ggagaggatc ctggctacgt tcctgttctg gctgatggac acatacgtgg tacagctgct 1680
   taggtcattc ttttacatca cagagagcac attccagaag aacaggctct tcttctaccg 1740
   taagagtgtg tggagcaagc tgcagagcat tggagtcagg caacaccttg agagagtgcg 1800
   gctacgggag ctgtcacaag aggaggtcag gcatcaccag gacacctggc tagccatgcc 1860
   catctgcaga ctgcgcttca tccccaagcc caacggcctg cggcccattg tgaacatgag 1920
   ttatagcatg ggtaccagag ctttgggcag aaggaagcag gcccagcatt tcacccagcg 1980
   tctcaagact ctcttcagca tgctcaacta tgagcggaca aaacatcctc accttatggg 2040
   gtcttctgta ctgggtatga atgacatcta caggacctgg cgggcctttg tgctgcgtgt 2100
   gcgtgctctg gaccagacac ccaggatgta ctttgttaag gcagatgtga ccggggccta 2160
   tgatgccatc ccccagggta agctggtgga ggttgttgcc aatatgatca ggcactcgga 2220
   gagcacgtac tgtatccgcc agtatgcagt ggtccggaga gatagccaag gccaagtcca 2280
   caagtccttt aggagacagg tcaccaccct ctctgacctc cagccataca tgggccagtt 2340
   ccttaagcat ctgcaggatt cagatgccag tgcactgagg aactccgttg tcatcgagca 2400
   gagcatctct atgaatgaga gcagcagcag cctgtttgac ttcttcctgc acttcctgcg 2460
   tcacagtgtc gtaaagattg gtgacaggtg ctatacgcag tgccagggca tcccccaggg 2520
   ctccagccta tccaccctgc tctgcagtct gtgtttcgga gacatggaga acaagctgtt 2580
   tgctgaggtg cagcgggatg ggttgctttt acgttttgtt gatgactttc tgttggtgac 2640
   gcctcacttg gaccaagcaa aaaccttcct cagcaccctg gtccatggcg ttcctgagta 2700
   tgggtgcatg ataaacttgc agaagacagt ggtgaacttc cctgtggagc ctggtaccct 2760
   gggtggtgca gctccatacc agctgcctgc tcactgcctg tttccctggt gtggcttgct 2820
   gctggacact cagactttgg aggtgttctg tgactactca ggttatgccc agacctcaat 2880
   taagacgagc ctcaccttcc agagtgtctt caaagctggg aagaccatgc ggaacaagct 2940
   cctgtcggtc ttgcggttga agtgtcacgg tctatttcta gacttgcagg tgaacagcct 3000
   ccagacagtc tgcatcaata tatacaagat cttcctgctt caggcctaca ggttccatgc 3060
   atgtgtgatt cagcttccct ttgaccagcg tgttaggaag aacctcacat tctttctggg 3120
   catcatctcc agccaagcat cctgctgcta tgctatcctg aaggtcaaga atccaggaat 3180
   gacactaaag gcctctggct cctttcctcc tgaagccgca cattggctct gctaccaggc 3240
   cttcctgctc aagctggctg ctcattctgt catctacaaa tgtctcctgg gacctctgag 3300
   gacagcccaa aaactgctgt gccggaagct cccagaggcg acaatgacca tccttaaagc 3360
   tgcagctgac ccagccctaa gcacagactt tcagaccatt ttggactaac cctgtctcct 3420
   tccgctagat gaacatgggc attgtagcct cagcactcct ggatccacgt cacaagaggg 3480
   actggtcagt tgtgaggcta ggtcatcctc caaacctctg tgtcatgggt ggtatgggag 3540
   attgtcccag tgccttgttt cctgtaacag gcttgatttc tttcctgatg ccctcaggga 3600
   ggcagatcct atccctttta gtggcaggga tccactagca ccagcacatg aggagtgcac 3660
   ccagtgcaca tgggcactgg gacagtggac aggtgtgaga ttcctgggcc ctggagtctt 3720
   ttcacaccta accatggagc ctgtcccagt acatcagagt gcctcggaga tgaaaaagga 3780
   catcgagcca gtgacctaaa ttacagcctg aatatactct gaattcatgt gactgcctta 3840
   gctacttctc tactgctgtg tagtaaaaca ccaagccaac ttataaaagc aggattttcc 3900
   tactggagca gcagctgaga gtttacatct tgatccataa gcacaaaagc acaagacaga 3960
   gagagagaga gagagagaga gagagagaga gagagagaga gagagagaga gagagagtca 4020
   gtcagtcagt ctaacaaata actaagaaag gtgaagggtg atgaagtcca cagggatcac 4080
   gctagggatg ttccatgcct tctctgaagc taagattcct tggcagcgtt tgacagtaac 4140
   catagtgggt acctactgag atcactataa agataaaata gggggaagcg tatttgtact 4200
   gaactggaaa aacatacaaa taaagagtaa atcatgg 4237
<210> 14
   <211> 3378
   <212> DNA
   <213> Rattus norvegicus
<400> 14
   atgccccgcg ctcctcgttg ccccgccgtg cgctctctac tgcgcagccg atatcgggag     60
   gtgtggccgc tggcgacctt tgtgcggcgc ctggggcttg agggcagtcg gcttgtgcaa    120
   cccggggacc cgaaggtctt ccgcacgttg gttgcccagt gcctagtgtg cgtgccctgg    180
   ggctcacagc cgccacctgc tgacctttcc ttccaccagg tgtcatccct gaaagagctg    240
   gtgtccaggg ttgtgcagaa actttgcgag cgcggtgaga ggaatgtgct ggcttttggc    300
   tttgcactgc ttaacggggc cagaggtggg cctcccatgg ccttcacgac cagcgtgcat    360
   agctacttgc ccaactcggt tactgagtcc ctgtgtgtca gtggtgcatg gatgctactg    420
   ttgagccgag tgggcgacga cctgctggtc tacctgctgt cgcactgtgc gctctacctg    480
   ctggtgcccc ccagctgtgc ctaccaggtg tgcgggtcac ccctgtacca aatttgtgcc    540
   accacggata cctggtcctc tgtgcccgct ggttacaggc ccactcgacc cgtgggcggg    600
   aatttcacta accttgggtc cgcacaccag atcaaaaaca gtggtcacca ggaagcacca    660
   aaaccccagg ccttgccatc acgaggtacg aagaggcttc tgagtctcac cagtacaaac    720
   gtgccttcag ctaagaaggc caggtttgaa cctgccctga gagtggataa gggaccccac    780
   aggcaggtgg taccaacccc atcaggcaaa acatgggcgc caagtcctgc tgcgtccccc    840
   aaggtgcctc ctgcagcgaa aaacttgtct ttgaaaggaa aggcatctga cccgagtctc    900
   tctgggtcgg tgtgctgtaa acacaagccc agctcctcgt ccctgctgtc atcaccaccc    960
   caagatgctg aaaagctcag gccattcact gagaccagac atttccttta ctccagggga   1020
   ggtggccaag aggagctaaa tccctcattc ctactcaaca gcctcccgcc tagcttgacc   1080
   ggggccagga gactggtgga gatcatcttt ctgggctcaa ggcctaggac atcaggacca   1140
   ttctgcagga cccgccgcct gccccgtcga tactggcaga tgcgacccct attccagcag   1200
   ctgctcatga accacgcaaa gtgccaatat gtcagattcc tccggtcgca ctgcagattt   1260
   cgaacagcaa accagcgggt gccggatgcc atggacacca gcccatccca cctcacgagt   1320
   ttgctccggt tacacagcag cccctggcag gtatacggct ttcttcgggc ctgcctccgc   1380
   gagctggtgc ctgccggtct ctggggcacc aggcacaatg agcgccgctt cttaaagaac   1440
   gtgaagaagt tcatctcgtt ggggaagtac gccaagctat ccctgcagga actgatgtgg   1500
   agggtgaaag tggaggactg ccactggctc cgcagcagcc cagagaagga cactgtccct   1560
   gccgcagagc accgtctgag ggagaggatc cttgccatgt tcctgttctg gctaatggac   1620
   acatatgtgg tacagctgct gaggtcattc ttctacatca cagagaccac gttccagaag   1680
   aaccgccttt tcttctaccg taagagtgtg tggagcaagc tgcagagcat tggaatcagg   1740
   caacagcttg agagagttca gctacgggaa ctgtcacaag aggaggtcaa gcatcaccag 1800
   gacacttggc tggccatgcc tatctgcaga ttgcgcttca tccccaagct caatggtctc 1860
   cggcccattg tgaacatgag ttatggcatg gacaccagag cttttggcaa aaagaagcag 1920
   acccagtgtt tcactcagag tctcaagact ttgttcagcg tgctcaacta cgagcggacc 1980
   aaacatccta accttatggg tgcttcagta ctgggtacga gtgacagcta caggatctgg 2040
   cggaccttcg tgctgcgtgt gcgtgctctg gaccagacac ccaggatgta ctttgttaag 2100
   gcagatgtga caggggccta tgatgccatc ccccaggaca agctcgtgga aattgtcgcc 2160
   aatataatca ggcgctcaga gagcatgtac tgtatccgcc agtatgcagt ggttcagaaa 2220
   gatagccaag gccaagtcca caagtccttc aggagacagg tctccaccct ctctgacctc 2280
   cagccataca tgggccagtt caccaagcat ctgcaggact cagatgccag tgcactgagg 2340
   aactctgttg tcatcgagca gagcatctcc atgaatgaga ctggcagtag cctgctccac 2400
   ttcttcctgc gctttgtccg tcacagtgtc gtgaagatcg atggcaggtt ctatgtgcaa 2460
   tgccagggca tcccccaggg ctccagcctg tccaccctgc tctgcagtct gtgtttcgga 2520
   gacatggaga acaagctgtt tgccgaggtg cagcaggacg gcttgctttt acgttttgtc 2580
   gatgactttc tgttggtgac acctcacctg gcccatgcaa aagcctttct cagcaccctg 2640
   gtccatggcg tgcccgagta tggctgcatg ataaacttgc agaagacagt ggtgaacttc 2700
   cctgtggaga ccggcgccct gggaggtgca gccccgcacc agctgcctgc tcactgcctg 2760
   tttccctggt gtggcttact gctggacact cggactttgg aagtattctg tgactactca 2820
   ggttacggac ggacctcaat taagatgagc ctcaccttcc agggtgtctc cagggccggg 2880
   aagaccatgc ggtacaagct cttgtcagtc ttgcggttga agtgtcatgg tctgtttcta 2940
   gacttgcagg tgaacagcct gcagacagtc tgcatcaata tatacaagat cttcctgctt 3000
   caggcctaca ggttccatgc atgtgtgatt cggcttccct ttggccagca tgttaggaag 3060
   aaccatgcat tctttctggg catcatctcc aacctagcat cctgctgcta cgccatcctg 3120
   aaggtcaaga atccaggagt gtcactaagg gccaagggtg cccctggctc ctttccgccc 3180
   gaggccacac gttggctctg ctaccaagcc ttcctgctca agctggctgc tcattctgtc 3240
   acctacaagt gtctcctggg acctcttagg acagcccaaa aacagctgtg ccggaagctc 3300
   ccagaggcaa caatgaccct ccttaagact gcagctgacc cagccctaag cacagatttt 3360
   cagaccattt tggactaa 3378
<210> 15
   <211> 1903
   <212> DNA
   <213> Homo sapiens
<400> 15
   cggcgtcccc ggggcccact cccgagcgca ggcgggcagc caggcgggcg gcgcggcgcg 60
   ggccggcagg aagcgtattc tgggcacggg gcgccgggcg ggccggctgc gccgagcggc 120
   agtggtggga taccacccaa ggcctcgcgc ggcgccgccc gtcgaggggc gggcggcggc 180
   gtagccactg ggccgtcgaa gagcgcagga ggccggtggg ccgggccggg ccgcgcggcg 240
   cagccatgcc tggctttacg tgctgcgtgc caggctgcta caacaactcg caccgggaca 300
   aggcgctgca cttctacacg tttccaaagg acgctgagtt gcggcgcctc tggctcaaga 360
   acgtgtcgcg tgccggcgtc agtgggtgct tctccacctt ccagcccacc acaggccacc 420
   gtctctgcag cgttcacttc cagggcggcc gcaagaccta cacggtacgc gtccccacca 480
   tcttcccgct gcgcggcgtc aatgagcgca aagtagcgcg cagacccgct ggggccgcgg 540
   ccgcccgccg caggcagcag cagcaacagc agcagcagca gcaacagcag caacagcagc 600
   agcagcagca acagcagcag cagcagcagc agcagcagca gtcctcaccc tctgcctcca 660
   ctgcccagac tgcccagctg cagccgaacc tggtatctgc ttccgcggcc gtgcttctca 720
   cccttcaggc cactgtagac agcagtcagg ctccgggatc cgtacagccg gcgcccatca 780
   ctcccactgg agaagacgtg aagcccatcg atctcacagt gcaagtggag tttgcagccg 840
   cagagggcgc agccgctgcg gccgccgcgt cggagttaca ggctgctacc gcagggctgg 900
   aggctgccga gtgccctatg ggcccccagt tggtggtggt aggggaagag ggcttccctg 960
   atactggctc cgaccattcg tactccttgt cgtcaggcac cacggaggag gagctcctgc 1020
   gcaagctgaa tgagcagcgg gacatcctgg ctctgatgga agtgaagatg aaagagatga 1080
   aaggcagcat tcgccacctg cgtctcactg aggccaagct gcgcgaagaa ctgcgtgaga 1140
   aggatcggct gcttgccatg gctgtcatcc gcaagaagca cggaatgtga actggtgccc 1200
   cggcagcctg ctggactccc agaccccatc cagccagggg accgcaggcc attgttgaac 1260
   tcctctatac tcctgggcac tggttgacag tactgaggct taaggcagct ggactctctt 1320
   gctggtgacc tggcatcctc aattgtttcc tcctgaagtg gaagctgggg ccttagactc 1380
   tgccctggtg acaccagcaa ttatgacttt gtctaccctt cctccccagt tattgttgca 1440
   gattctggtt aagcagaggc ttcagaacca ctgaacttga aacttaccct ctagggatgc 1500
   aggtgggatg tccagggact ataggtttgg gaaaaccata ccttaaggtt ggtcagcagt 1560
   cagacaactc taatgtgtgt agtgataaga gattcaagta acatcagttc tcctcctttt 1620
   catgcttttc cttcccaggt gcagcctgtg attctgatgg ggactggtaa atctgtgcct 1680
   ctgcctccta ggacttattt tcccaggagg ccatttacaa ggggatctgg atgacctgct 1740
   gatggagatc cagcttgcca gggacttagg tttatcctgt tttgtttgct actggttaca 1800
   aattctattt tctgtacaat tagtcagact aaagttttca ctgtgtttgt ttggcaaaac 1860
   aaattaaaca aaaagtaagg tttttaaaaa aaaaaaaaaa aaa 1903
<210> 16
   <211> 1832
   <212> DNA
   <213> Mus musculus
<400> 16
   gaagcgcagg cggacggccg ggtgggtagc agcggcgcgg gcggtccgca accatattct 60
   gggcacgggg cctcggcccg ggacgactgc gccgggcggc aatagtgaga ggcctctgag 120
   agcctcgcgc ggcgccgccc gtcgaggagc tgagacaggg gccagacccg gccgtcgaag 180
   agctcgagag gccggtgggc cgggccgcgc gtcgcagcca tgcctggctt tacgtgctgc 240
   gttccgggct gctacaacaa ttcacaccgg gacaaggcgc tgcacttcta cacgtttccc 300
   aaggacgctg agttgcggcg cctctggctc aagaacgtgt cccgtgctgg cgtcagtggg 360
   tgcttctcca ccttccaacc caccaccggc caccgtctct gcagcgtcca ctttcagggc 420
   ggccgcaaga cctacacggt gcgcgttccc accattttcc cgctgcgtgg cgtcaatgag 480
   cgcaaagtag ctcggagacc tgcgggagct gcggcagccc gccgtaggca gcagcagcag 540
   caacaacaac agcagcagca gcaacagcag cagctgcaac agcagcagcc gtctccgtcc 600
   tcctccactg cccagaccac ccagttgcag ccgaacctgg tgtctgcctc tgcagctgtg 660
   cttcttacgc ttcaggccgc cgtagacagc aaccaggctc cgggatccgt ggttcccgtg 720
   tccacgactc cctcgggaga tgatgtgaag cccatcgacc tcacagtgca agtcgagttt 780
   gcagctgctg aaggggcagc cgccgctgcc gccgcctcag agctagaggc tgctacggct 840
   gggctggagg ccgctgagtg cactctgggc cctcagctgg tggtggtagg ggaagagggc 900
   ttccctgaca ctggctctga ccactcgtac tccttgtcct cgggtaccac ggaggaggag 960
   ctcctgcgca agctgaacga gcagcgggac atcttggccc tgatggaagt gaagatgaag 1020
   gagatgaagg gtagcatccg ccatctgcgt ctcaccgagg ccaagctccg tgaagaactt 1080
   cgagagaagg atcgtctgct tgccatggct gtcatccgta agaagcacgg catgtgaatg 1140
   gttcccccca gaaacctgca gaattcgtga ctccttccag cccaaggaat cctcaggcaa 1200
   atgttgtact ccccagtatt cctgttgaca gtgccgaggt ttaggacagc gggactccag 1260
   ttggtcagtt ggcaccttct ttcgtctcct cgtgaagtaa gagttgggga cttcagaatc 1320
   tggtgacacc agcagttatg actttgtctc tcactcccca gtttatgttg cagattctgg 1380
   ttatacacag gcttcagaac cactgaactt ggaacttacc ctggaggggg tgcagatgga 1440
   actcttaagg gactgtgggt ttgaggaaac cacttcttca ggttggccag caatcagaga 1500
   cagctttgct gtgtgtagtg ataagagatc tcagtaacat cgtttctcct ttccatgcta 1560
   cccggttcag gtgcagtctg tgattctgat ggggactagc tgatctgtgc ctctgtctct 1620
   taggacctct ctacccagaa ggtcatttat gagagggctc tgggtgactt gctgatggag 1680
   atccagctca ccagggactt aggtttatct cgttatgttt gctactggtt acaaattcta 1740
   ttttctgtac aattagactg aagttttcac tgtttggcaa aacaaattaa acaaaaaagt 1800
   aaggttttta aaaaaaaaaa aaaaaggcca ca 1832
<210> 17
   <211> 55
   <212> DNA
   <213> Escherichia coli
<400> 17
   tctagagcgg ccgcccggcg ggtcgccacc atgagtgtgg atccagcttg tcccc 55
<210> 18
   <211> 36
   <212> DNA
   <213> Escherichia coli
<400> 18
   catgcaacct gaagacgtgt gaagactagt atcgat 36
<210> 19
   <211> 51
   <212> DNA
   <213> Escherichia coli
<400> 19
   tctagagcgg ccgcccggcg ggtcgccacc atggctgtca gcgacgcgct g 51
<210> 20
   <211> 46
   <212> DNA
   <213> Escherichia coli
<400> 20
   ccacctcgcc ttacacatga agaggcattt ttaaaagctt atcgat 46
<210> 21
   <211> 50
   <212> DNA
   <213> Escherichia coli
<400> 21
   tctagaggat ccccggcggg tcgccaccat ggcggccgcg ggaattcgat 50
<210> 22
   <211> 39
   <212> DNA
   <213> Escherichia coli
<400> 22
   ggcacactgc ccctctcaca catgtgaaag cttatcgat 39
<210> 23
   <211> 51
   <212> DNA
   <213> Escherichia coli
<400> 23
   tctagagcgg ccgcccggcg ggtcgccacc atggcgggac acctggcttc g 51
<210> 24
   <211> 37
   <212> DNA
   <213> Escherichia coli
<400> 24
   gggctctccc atgcattcaa actgaggatc catcgat 37
<210> 25
   <211> 50
   <212> DNA
   <213> Escherichia coli
<400> 25
   tctagagcgg ccgcccggcg ggtcgccacc atgccgcgcg ctccccgctg 50
<210> 26
   <211> 41
   <212> DNA
   <213> Escherichia coli
<400> 26
   caaaatccca agcgtggcgc tgcccttaaa agcttatcga t 41
<210> 27
   <211> 43
   <212> DNA
   <213> Escherichia coli
<400> 27
   tctagagcgg ccgcccggcg ggtcgccacc atggtgagca agg 43
<210> 28
   <211> 38
   <212> DNA
   <213> Escherichia coli
<400> 28
   ctcggcatgg acgagctgta caataaaagc ttatcgat 38

## Description

The present invention relates to a process for preparing reprogrammed cells, in particular for preparing pluripotent and multipotent stem cells and to the pluripotent and multipotent stem cells prepared by said processes.

Aging - whether in vivo during the life course or in vitro during scale up - affects cells, in particular stem cells. For example, aged cells show increased apoptosis rates, decreased differentiation and proliferation capacity, and decreased therapeutic effectiveness. During aging, stem cells often differentiate into a less pliable, less proliferative form. Cell aging represents a problem in particular when using cells from elderly patients/donors as starting material for cell cultures, when expanding cells in vitro and when using cells that have a limited differentiation capacity for therapy or research.

One approach to dedifferentiate aged cells is to create so called Induced Pluripotent Stem Cells (IPS). The current state of the art foresees to use a viral transfer of transcription factors to induce increased differentiation potency in cells and to rejuvenate them. This, however, is potentially dangerous because a viral transfer permanently changes the genome of the cell and also might lead to complications because of the viral parts left behind in the cell genome. Moreover, viral integration into the genome is known to increase cancer risk in cells. Essentially the same applies to the use of plasmids for reprogramming cells (Okita et al., 2008, Science. 2008 Nov 7;322(5903):949-53. Generation of mouse induced pluripotent stem cells without viral vectors).

Another approach employs contacting cells to be reprogrammed with extracts of human embryonic stem cells for reprogramming cells. This approach, however, is ethically and practically problematic.

Some other approaches use chemical factors to dedifferentiate cells (e.g. US 2007/0254884 A1, US 2007/0020759 A1), but these factors are not efficacious in fully reprogramming cells to a pluripotent state by themselves.

WO 2008/087442 A discloses methods for inducing the reprogramming of a differentiated cell by adding Nanog MRNA to a non-Nanog containing Xenopus oocyte and introducing the extract of that oocyte into a cell to be reprogammed. Jaenisch et al., Cell 133 (4), February 2008:567-582, describes the reprogramming of somatic cells by nuclear transplantation of a somatic nucleus into an enucleated egg and by means of retroviruses to introduce nucleic acid molecules into a cell to be reprogrammed.

Zuk et al. "Human adipose tissue is a source of multipotent stem cells", Molecular Biology of the Cell, December 2002, Vol. 13, 4279-4295 disclosed that adipose tissue contains multipotent stem cells, in particular mesenchymal stem cells that expressed multiple CD marker antigens that are characteristic for such multipotent cells.

Thus, the technical problem underlying the present invention is to provide improved methods to reprogram cells, in particular to dedifferentiate cells, which overcome the above-identified disadvantages, in particular allow in an easy, efficient and safe way the provision of reprogrammed, in particular dedifferentiated cells, preferably displaying multipotent, most preferably pluripotent, stem cell character.

The present invention solves its technical problem by the provision of a process for preparing reprogrammed cells in vitro, comprising the process steps, preferably in the given order, a) providing cells to be reprogrammed; b) introducing, preferably transfecting, mRNA molecules capable of reprogramming the cells into the cells provided in step a), wherein the reprogramming mRNA molecules are coding for at least one protein selected from the group consisting of: Ronin, Oct4, Klf4, Sox2, Nanog and TERT; and c) culturing the cells obtained in step b) in a cell culture medium and under a condition suitable to allow translation of the introduced, preferably transfected reprogramming mRNA molecules so as to obtain reprogrammed cells.

Thus, the present invention foresees a process which is able to prepare reprogrammed cells without the need to permanently change the genome of the cell and without the need to use viruses to transfer transcription factors or the like into the cell. The present invention also is advantageous in so far, as for reprogramming cells no extracts of human embryonic stem cells or cost intensive and potentially dangerous chemical factors are needed. Thus, ethical concerns, genetic stability problems and cancer risks are reduced or even avoided. In contrast, the present invention provides the advantageous teaching to introduce, preferably transfect, specific mRNA molecules, in the following termed "reprogramming mRNA molecules", into the cells, wherein these reprogramming mRNA molecules are able to reprogram the mRNA recipient cells, preferably the cells being transfected therewith, into a dedifferentiated status. Advantageously, the mRNA molecules introduced, preferably transfected, according to the present invention will not integrate into the recipient's genome and therefore do not pose a risk of cancer or genetic instability. The invention therefore foresees a process for preparing one or more reprogrammed cells in vitro wherein said process does not include any cloning amplification or proliferation of the mRNA recipient cells. The present invention also foresees a process for preparing one or more reprogrammed cells in vivo, that is in a living organ, a living organism or animal, more particular a mammal.

In the context of the present invention the term "reprogramming" preferably means remodelling, in particular erasing and/or remodelling, epigenetic marks of a cell such as DNA methylation, histone methylation or activating genes by inducing transcription factor signal systems as for oct4. In particular, the reprogramming of the present invention provides at least one dedifferentiated and/or rejuvenated cell, in particular provides a cell having the characteristic of a multipotent, in particular pluripotent stem cell. Thus, in case the cells to be reprogrammed are cells which already have a multipotent or pluripotent character, the present invention is able to maintain these cells by the reprogramming of the present invention in their multi- or pluripotent state for a prolonged period of time. In case the cells to be reprogrammed are in an aged or differentiated state, the present invention allows the dedifferentiation into a multipotent or pluripotent stem cell. In a particularly preferred embodiment, multipotent cells may be reprogrammed to become pluripotent cells.

In particular, the term "reprogrammed cells" designates cells which have been changed to have a higher differentiation and/or proliferation potential. Also, in a preferred embodiment the reprogramming of a somatic differentiated cell toward a multipotent stem cell or "young" differentiated cell is termed reprogramming and the product is called a reprogrammed cell.

In particular, the present process allows it to enrich immature stem cells, preferably showing a high telomerase activity, several multipotent, preferably pluripotency, markers and an increased secretion of growth factors. The present invention provides the advantage that aged stem cells can be expanded for a longer period of time and with a higher stem cell yield. Further, stem cell lines and tissue engineered construct drafts made or derived from cells reprogrammed by the present process show a reduced minimal immunological risk, in particular if the patient's own cells are used as a source for the reprogramming. Thus, the present invention provides means and methods to prepare in particular pluripotent stem cells, to provide multipotent stem cells, to provide means and methods for an increased expansion of cells or stem cells in vitro and for the rejuvenation of aged cells or aged stem cells for tissue engineering or cell therapies.

In particular, the present invention foresees, preferably in a first step, to provide cells to be reprogrammed. Preferably, the cells to be reprogrammed may be adult or neonatal cells, but are not limited thereto. Preferably, the cells to be reprogrammed are adult or neonatal undifferentiated cells. All of these cells may, in a preferred embodiment, be cell lines, immortalized cells, cells kept in cell culture, isolated cell populations, preferably cells isolated from a donor, either a living or dead donor, or cells isolated from the environment. Cell types which can be reprogrammed according to the present invention potentially are all cell types, and are in preferred instances fibroblasts, hepatocytes, cardiac cells, cardiomyocytes, nerve cells, chondrocytes, osteoblasts, adipocytes, myoblasts, hepatoblasts, hepatic stem cells, insulin producing cells, neural stem cells, cardiomyogenic cells, dermatocytes, keratinocytes, pancreatic cells, monocytes, epithelial cells or mesenchymal stem cells (MSC). Preferably, the cells may be mammalian cells, in particular human cells or animal cells, preferably ovine, horse, ape or in particular rodent cells, preferably hamster cells, mouse cells or rat cells. The cells may also be fish, reptile, avian, amphibian or insect cells. Preferably, the cells to be reprogrammed are lineage-committed cells. In a further preferred embodiment the cells, in particular the cells of the above identified origin, are stem cells, in particular post-natal stem cells or non-embryonic stem cells.

A suitable and preferred stem cell source like mesenchymal stem cells is a tissue within the human or animal body which comprises the stem cells for use in the present invention, optionally together with other cell types. Preferably, the suitable stem cell source is bone marrow, both adult and fetal, cytokine or chemotherapy mobilized peripheral blood, fetal liver, umbilical cord blood, and spleen, both adult and fetal, more preferably the stem cell source is adult bone marrow or umbilical cord blood, most preferably the stem cell source is bone marrow. Bone marrow cells may be obtained from any known source, including, but not limited to, ilium, sternum, tibiae, femora, spine or other bone cavities. Preferably, the stem cells are isolated from a mammalian organism such as human, mouse or rat, and more preferably the stem cells are isolated from a human organism.

The term "isolated cell population" is intended to mean that the cells are not in contact with other cells with which they are usually in contact within the body of the mammal or in a tissue sample obtained directly, i.e. without purification or enrichment step, from the mammal. A "cell population" according to the present invention may comprise not only cells of one cell type such as fibroblasts or mesenchymal stem cells as defined for example by the expression of a specific combination of surface markers, but also a mixture of cells of different cell types which show different combinations of surface markers.

The cells harvested from said sources may be used directly for transfection or may be cryoconserved by freezing them at a temperature from about - 196°C to about - 130°C.

The cells to be reprogrammed receive, according to the present invention, in a second step at least one species of reprogramming mRNA molecules, in particular linear and isolated mRNA molecules, selected from the group of mRNA molecules encoding Ronin, mRNA molecules encoding Oct4, mRNA molecules encoding Klf4, mRNA molecules encoding Sox2, mRNA molecules encoding Nanog and mRNA molecules encoding TERT. Ronin, Oct4, Klf4, Sox2, Nanog and TERT are proteins with the ability to reprogram cells. In the context of the present invention these proteins are termed "reprogramming proteins" and are characterised by their ability to reprogram target cells and therefore by their regulatory function in terms of determining the cell fate, in particular being able to dedifferentiate a target cell and/or maintain a cell in a dedifferentiated state, preferably by being transcription factors.

Differentiation behaviour of cells and state of differentiation can be detected according to the methods set forth below. However any method for determining the differentiation state or potency of a cell known the skilled person arc applicable as well.

In a preferred embodiment, the cell to be reprogrammed is transfected with one or more reprogramming mRNA molecules according to the present invention. In another preferred embodiment the cell to be reprogrammed is supplemented with one or more reprogramming mRNA molecules according to the present invention. In another preferred embodiment the cell to be reprogrammed is injected with one or more reprogramming mRNA molecules according to the present invention.

The present invention thus foresees to use one or more of mRNA molecules encoding Ronin, Oct4, Klf4, Sox2, Nanog or TERT. These mRNA molecules may, in a preferred embodiment, be molecules having the wild-type mammalian, in particular human, animal, preferably rodent, more preferably mouse, hamster or rat or any other animal nucleotide sequence. In a preferred embodiment of the present invention the reprogramming mRNA molecule is selected from the group consisting of mRNA nucleotide sequence molecules being encoded by any one of the DNA sequences given under SEQ ID No. 1 to 16.

Of course, the invention also foresees to use mRNA molecules, whose sequence is a functional equivalent to the polynucleotide sequence given above. In the context of the present invention a functional equivalent is a nucleotide sequence molecule, which either codes with a different nucleotide sequence exactly the same protein as the mRNA sequence encoded by any one of SEQ ID No. 1 to 16 or is a mRNA sequence, which encodes a protein with a different amino acid sequence but with same or similar function, in particular being able to reprogram the cells according to the present invention.

In a particularly preferred embodiment of the present invention the functional equivalent of the mRNA molecules is a polynucleotide with a homologous sequence to the wild-type mRNA polynucleotide as encoded by the DNA sequence in any one of SEQ ID No. 1 to 16. In a preferred embodiment of the present invention the degree or percentage of homology is at least 50, 60, 70, 80, 90, 95, 99 or 100%. In a furthermore preferred embodiment a functional equivalent of the mRNA molecules as given in any one of SEQ ID No. 1 to 16 is a mRNA sequence which is encoded by a DNA sequence being substantially complementary to the sequences of any one of SEQ ID No. 1 to 16 or to its complementary, preferably substantially, complementary sequence. In a furthermore preferred embodiment the functional equivalent of the mRNA molecule as encoded by any one of the DNA sequences of SEQ ID No. 1 to 16 is a RNA molecule being encoded by a DNA sequence which is able to hybridize under stringent or reduced stringent conditions to any one of the polynucleotides given in SEQ ID No. 1 to 16 or to its complementary, preferably substantially, complementary sequence.

In the context of the present invention the term "mRNA molecule" is meant to refer to a linear polymer of ribonucleotide molecules, which is single-stranded and serves as a template for protein synthesis.

Polynucleotides have "homologous" sequences if the sequence of nucleotides in the two sequences is the same when aligned for maximum correspondence as described herein. Sequence comparison between two or more polynucleotides is generally performed by comparing portions of the two sequences over a comparison window to identify and compare local regions of sequence similarity. The comparison window is generally from about 20 to 200 contiguous nucleotides.

The "percentage of sequence homology" for polynucleotides, herein referred to be 50, 60, 70, 80, 90, 95, 98, 99 or 100 percent sequence homology, may be determined by comparing two optimally aligned sequences over a comparison window, wherein the portion of the polynucleotide sequence in the comparison window may include additions or deletions (i.e. gaps) as compared to the reference sequence (which does not comprise additions or deletions) for optimal alignment of the two sequences. The percentage is calculated by: (a) determining the number of positions at which the identical nucleic acid base occurs in both sequences to yield the number of matched positions; (b) dividing the number of matched positions by the total number of positions in the window of comparison; and (c) multiplying the result by 100 to yield the percentage of sequence homology. Optimal alignment of sequences for comparison may be conducted by computerized implementations of known algorithms, or by inspection. Readily available sequence comparison and multiple sequence alignment algorithms are, respectively, the Basic Local Alignment Search Tool (BLAST) (Altschul, S.F. et al. 1990. J. Mol. Biol. 215:403; Altschul, S.F. et al. 1997. Nucleic Acid Res. 25:3389-3402) and ClustalW programs both available on the internet. Other suitable programs include GAP, BESTFIT and FASTA in the Wisconsin Genetics Software Package (Genetics Computer Group (GCG), Madison, WI, USA).

As used herein, "substantially complementary" means that two nucleic acid sequences in question have at least about 65%, preferably about 70%, more preferably about 80%, even more preferably 90%, and most preferably about 98%, sequence complementarity to each other. This means that the DNA sequence coding the functional equivalent and the polynucleotide given in any of SEQ ID No. 1 to 16 or its complement must in a preferred embodiment exhibit sufficient complementarity to hybridise under stringent conditions. A substantially complementary sequence is preferably one that has sufficient sequence complementarity to the nucleotide sequence in question to result in binding.

The term "primer" as used herein refers to an oligonucleotide which is capable of annealing to the amplification target allowing a DNA polymerase to attach thereby serving as a point of initiation of DNA synthesis when placed under conditions in which synthesis of primer extension product which is complementary to a nucleic acid strand is induced, i.e., in the presence of nucleotides and an agent for polymerization such as DNA polymerase and at a suitable temperature and pH. The (amplification) primer is preferably single stranded for maximum efficiency in amplification. Preferably, the primer is an oligodeoxy ribonucleotide. The primer must be sufficiently long to prime the synthesis of extension products in the presence of the agent for polymerization. The exact lengths of the primers will depend on many factors, including temperature and source of primer.

A "pair of bi-directional primers" as used herein refers to one forward and one reverse primer as commonly used in the art of DNA amplification such as in PCR amplification.

The terms "stringency" or "stringent hybridization conditions" refer to hybridization conditions that affect the stability of hybrids, e.g., temperature, salt concentration, pH, formamide concentration and the like. These conditions are empirically optimised to maximize specific binding and minimize nonspecific binding of an oligo- or polynucleotide to its target nucleic acid sequence. The terms as used include reference to conditions under which an oligo- or polynucleotide will hybridise to its target sequence, to a detectably greater degree than other sequences (e.g. at least 2-fold over background). Stringent conditions are sequence dependent and will be different in different circumstances. Longer sequences hybridise specifically at higher temperatures.

Generally, stringent conditions are selected to be about 5 DEG C (degree celcius) lower than the thermal melting point (Tm) for the specific sequence at a defined ionic strength and pH. The Tm is the temperature (under defined ionic strength and pH) at which 50% of a complementary target sequence hybridises to a perfectly matched oligo- or polynucleotide. Typically, stringent conditions will be those in which the salt concentration is less than about 1.0 M Na <+> ion, typically about 0.01 to 1.0 M Na <+> ion concentration (or other salts) at pH 7.0 to 8.3 and the temperature is at least about 30 DEG C for short probes or primers (e.g. 10 to 50 nucleotides) and at least about 60 DEG C for long probes or primers (e.g. greater than 50 nucleotides). Stringent conditions may also be achieved with the addition of destabilizing agents such as formamide.

Exemplary low stringent conditions or "conditions of reduced stringency" include hybridization with a buffer solution of 30% formamide, 1 M NaCl, 1% SDS at 37 DEG C and a wash in 2x SSC at 40 DEG C. Exemplary high stringency conditions include hybridization in 50% formamide, 1 M NaCl, 1% SDS at 37 DEG C, and a wash in 0.1 x SSC at 60 DEG C. Hybridization procedures are well known in the art and are described in e.g. Ausubel et al, Current Protocols in Molecular Biology, John Wiley & Sons Inc., 1994.

Thus, the present invention foresees to introduce, preferably transfect, specific reprogramming mRNA molecules into the cells, which mRNA molecules once introduced, preferably transfected, in the cells are able to be translated into so called reprogramming proteins which can turn on specific genes in the cells, in particular multipotency and/or the pluripotency genes in the cell. The mRNA molecule used according to the present invention is preferably a linear molecule having a poly(A) tail, most preferably produced by in vitro transcription. Thus, in a preferred embodiment the mRNA molecules are produced by in vitro transcription, in particular using bacterial systems. In a particular preferred embodiment the DNA sequences of the reprogramming proteins are cloned into plasmids and amplified in bacteria, for instance E. coli.

In a further preferred embodiment, the plasmids are then isolated from the bacteria, are linearised and subjected to restriction digestion. In a further preferred embodiment cDNA prepared by said method is transcribed into mRNA, which in turn is incubated to destroy cDNA residues and to obtain mRNA molecules to be introduced into the cells.

Since the introduced reprogramming mRNA molecules and the proteins translated therefrom are over the time being degraded in the cells, they cannot permanently integrate into the genome of the cell providing some of the advantageous features of the present invention.

In a preferred embodiment of the present invention the present teaching foresees to introduce, preferably transfect, the reprogramming mRNA molecules into the cell by electroporation, by lipofection, by injection, by magnetofection, by particle bombardment, gene gun, or by any other method known in the art suitable to introduce mRNA molecules into a target cell.

In a particular preferred embodiment the present invention further foresees in its step c) to culture the cells into which the reprogramming mRNA molecules have been introduced in a cell culture medium and under a condition suitable to allow translation of the transfecting reprogramming mRNA molecules, so as to obtain the reprogrammed cells.

In a particularly preferred embodiment of the present invention the present process therefore consists of the above-identified steps a), b) and c), preferably in this order. Accordingly, the present invention excludes any further process steps, in particular any further substantial process step, in particular any intervening or subsequent process steps. Thus, the present invention provides its advantages in a simple and cost-effective way.

In a particularly preferred embodiment the cell culture system is a cell culture system, comprising a cell culture medium, preferably in a culture vessel, in particular a cell culture medium supplemented with at least one so called "inducing substance", which is a substance suitable and determined for protecting the cells from in vitro aging and/or inducing in an unspecific or specific reprogramming. In a particularly preferred embodiment an inducing substance according to the present invention is a substance selected from the group consisting of reversine, resveratrol, selenium, a selenium-containing compound, EGCG ((-)-epigallocatechin-3-gallate), valproic acid and salts of valproic acid, in particular sodium valproate.

In a particularly preferred embodiment the at least one inducing substance is present in the cell culture medium used in step c) of the present process in a concentration from 0.001 to 100 µM, preferably from 0.005 to 50 µM.

In a particularly preferred embodiment the present invention foresees to use a concentration of reversin from 0.5 to 10 µM, preferably of 1 µM. In a furthermore preferred embodiment the present invention foresees to use resveratrol in a concentration of 10 to 100 µM, preferably 50 µM. In a furthermore preferred embodiment the present invention foresees to use selenium or a selenium containing compound in a concentration from 0.05 to 0.5 µM, preferably of 0.1 µM. In a furthermore preferred embodiment the present invention foresees to use EGCG in a concentration from 0.001 to 0.1 µM, preferably of 0.01 µM. In a furthermore preferred embodiment the present invention foresees to use valproic acid or sodium valproate in a concentration from 1 to 10 µM, in particular of 5 µM.

The present invention foresees in a furthermore preferred embodiment culturing the cells obtained in step b) in a cell culture medium, wherein the cell culture medium comprises, optionally in combination with the inducing substance as specified above, at least one transient proteolysis inhibitor. The use of at least one proteolysis inhibitor in the cell culture medium of the present invention increases the time the reprogramming proteins derived from the mRNA or any endogenous genes will be present in the cells and thus facilitates in an even more improved way the reprogramming by the transfected mRNA derived factors. The present invention uses in a particularly preferred embodiment as a transient proteolysis inhibitor a protease inhibitor, a proteasome inhibitor and/or a lysosome inhibitor. In a particularly preferred embodiment the proteasome inhibitor is selected from the group consisting of MG132, TMC-95A, TS-341 and MG262.

In a furthermore preferred embodiment the protease inhibitor is selected from the group consisting of aprotinin, G-64 and leupeptin hemisulfate. In a furthermore preferred embodiment the lysosomal inhibitor is ammonium chloride.

In a furthermore preferred embodiment the present invention also foresees a cell culture medium comprising at least one transient inhibitor of mRNA degradation. The use of a transient inhibitor of mRNA degradation increases the half-life of the reprogramming factors as well.

In a furthermore preferred embodiment of the present invention a condition suitable to allow translation of the transfected reprogramming mRNA molecules in the cells is an oxygen content in the cell culture medium from 0.5 to 21 %, preferably from 1 to 20%, more preferably from 5 to 19% and particularly preferred from 10 to 18%. More particularly, and without wishing to be bound to theory, oxygen is used to further induce or increase Oct4 by triggering Oct4 via Hif1a.

In a preferred embodiment conditions that are suitable to support reprogramming of the cells by the mRNA molecules in the cells are selected; more particularly, these conditions require a temperature from 30 to 38°C, preferably from 31 to 37°C, most preferably from 32 to 36°C.

The glucose content of the medium is in a preferred embodiment of the present invention below 4.6 g/l, preferably below 4.5 g/l, more preferably below 4 g/l, even more preferably below 3 g/l, particularly preferably below 2 g/l and most preferably it is 1 g/l. DMEM media containing 1 g/l glucose being preferred for the present invention are commercially available as "DMEM low glucose" from companies such as PAA, Omega Scientific, Perbio and Biosera. More particularly, and without wishing to be bound to the theory, high glucose conditions adversely support aging of cells (methylation, epigenetics) in vitro which may render the reprogramming difficult.

In a furthermore preferred embodiment of the present invention the cell culture medium contains glucose in a concentration from 0.1 g/I to 4.6 g/l, preferably from 0.5 g/l to 4,5 g/l and most preferably from 1 g/I to 4 g/l.

The terms "cell culture" and "culturing of cells" refer to the maintenance and propagation of cells and preferably human, human-derived and animal cells in vitro.

"Cell culture medium" is used for the maintenance of cells in culture in vitro. For some cell types, the medium may also be sufficient to support the proliferation of the cells in culture. A medium according to the present invention provides nutrients such as energy sources, amino acids and inorganic ions. Additionally, it may contain a dye like phenol red, sodium pyruvate, several vitamins, free fatty acids, antibiotics, anti-oxidants and trace elements.

For culturing the stem cells according to the present invention any standard medium such as Iscove's Modified Dulbecco's Media (IMDM), alpha-MEM, Dulbecco's Modified Eagle Media (DMEM), RPMI Media and McCoy's Medium is suitable before reprogramming. Once the cells have been reprogrammed, they can in a preferred embodiment be cultured in embryonic stem cell medium.

Preferably, the medium additionally comprises one or more additives selected from the group consisting of vitamin D3 (1,25-dihydroxyvitamin D3, calcitriol), resveratrol (trans-3, 4', 5-trihydroxystilbene), reversine (2-(4-morpholinoahilino)-N6-cyclohexyladenine), vitamin E (RRR-α-tocopherol), valproic acid (dekapene, valproate, valrelease), EGCG (epigallocatechin-3-gallat) and selenium. Preferably, two of the afore-mentioned additives are present, more preferably three of the afore-mentioned additives are present, even more preferably four of the afore-mentioned additives are present, particularly preferably five of the afore-mentioned additives are present and most preferably all of the aforementioned additives are present.

These media are well-known to a person skilled in the art and may be purchased from companies such as Cambrex, Invitrogen, Sigma-Aldrich or Stem Cell Technologies.

The medium may in a particular embodiment further comprise a serum component such as horse serum, human serum or fetal calf serum (FCS). Preferably, the medium contains FCS. If present, the serum is present in a concentration of 1-20%, preferably of 3-18%, more preferably of 5-15%, even more preferably of 8-12% and most preferably of 10%. Alternatively the serum component may be replaced by any of several standard serum replacement mixtures which typically include insulin, albumin and lecithin or cholesterol.

A "culture vessel" is any vessel which is suitable for growing cells in a culture medium, in particular selected from, but not limited to, agar, matrigel and collagen, either in fluid phase or adhered to an interior surface of the container. Types of such specialized vessels include roller bottles, spinner flasks, petri dishes and tissue flasks. Culture vessels are designed to be incubated in temperature, humidity and gas controlled environments to facilitate maximum cell or tissue growth. Generally, a layer of cell culture medium or agar covers the growing surface. The portion of the vessel not utilized as a growing surface encloses the interior gaseous environment which surrounds the cell culture. Cells, tissues, microorganisms and the like typically are introduced into the interior of cell culture vessels through an opening in the vessel. After introduction of the cells the opening may be closed such that the cells are not in contact with the environment during the culturing of the cells.

In a preferred embodiment of the present invention, the culture vessel is treated for tissue culture, which means that the surface of the culture vessel is treated such that cells which usually grow in an adherent state grow adherently, but cells which grow in suspension do not adhere or adhere only loosely. Such treatment may involve the irradiation of the vessel or the coating of the vessel, for example with a special plastic, polymer or nanostructure or with proteins of the extracellular matrix. Such vessels can comprise tissue culture dishes and tissue culture flasks and are available from different suppliers such as Becton Dickinson, Greiner, Sigma and TPP.

The present invention also relates to a process for the production of cells exhibiting a multipotent, preferably a pluripotent, stem cell character, wherein a process according to the above is carried out.

The present invention also relates to a process for improving the expansion of stem cells in vitro, wherein a process according to the present invention is carried out. Thus, the present invention provides the advantage of prolonging expansion of stem cells, since the present invention keeps the cells to be expanded in a dedifferentiated status.

The present invention also relates to a process for the rejuvenation of aged cells, wherein a process according to the present invention is carried out.

The present invention also relates to a process for inducing the dedifferentiation of cells, wherein a process according to the present invention is carried out and wherein in particular the cells to be reprogrammed are differentiated cells, in particular lineage-committed cells.

The present invention also relates to a process for reprogramming recipient cells of a mammal in vivo comprising introducing reprogramming mRNA molecules into the recipient cells of the mammal wherein the reprogramming mRNA molecules are encoding at least one reprogramming protein selected from the group consisting of Ronin, Oct4, Klf4, Sox2, Nanog and TERT. Thus, the present invention also relates to an in vivo method for reprogramming recipient cells of a mammal which employ the reprogramming mRNA molecules as identified above for the in vitro embodiment of the present invention. The present invention relating to the process for reprogramming recipient cells of a mammal in vivo thereby foresees to introduce the reprogramming mRNA molecules directly into at least one cell of a recipient mammal that means into at least one recipient cell by suitable means such as gene gun insertion. Such a process allows the production of a mammal in particular a human being which has at least one reprogrammed cell. Thus, this embodiment of the present invention provides terrifically improved therapeutic application, in particular in the regenerative medicine and/or in replacement therapy. Thus, the present invention relates to a method of treating a mammal, in particular a human being wherein reprogramming mRNA molecules are introduced into at least one cell of the recipient mammal and wherein the reprogramming mRNA molecules are encoding at least one reprogramming protein selected from the group consisting of Ronin, Oct4, Klf4, Sox2, Nanog and TERT.

The present invention also relates to a multipotent, in particular a pluripotent, stem cell prepared according to any one of the methods of the present invention. Thus, the reprogrammed cells according to the present invention are preferably multipotent, in particular pluripotent stem cells. In a preferred embodiment the cells obtained according to the present invention are distinguished from the cells used as a starting material in step a) of the present invention by lack of one or more markers. These cells may in a preferred embodiment be also characterized by a particular methylation pattern, by the expression of oct4, sox2, nanog, hTERT or klf4. Expression of oct4, which is not found in somatic cells like fibroblasts, becomes expressed after transfecting mRNA (klf4, sox2 and oct4) in human fibroblasts, by improved growth potential; and by increased differentiation potential. For pluripotency, i.e. the formation of embryoid bodies, production of cells of all three germ layers in vitro and in vivo. Examples thereof are illustrated in the accompanying figures and example.

The reprogrammed cells obtained by the present processes may preferably be characterized by the expression or non-expression of certain surface markers. These surface markers are usually given a cluster of differentiation (CD) designation which describes groups of immunophenotypical surface characteristics of cells. Usually CD molecules are membrane-bound glycoproteins which are recognized by a cluster of monoclonal antibodies that display the same cellular reactivity. These surface molecules may for example be detected by means of a flow cytometer such as a fluorescence-activated cell sorter (FACS) manufactured by companies such as Becton Dickinson. Methods to identify cells according to the present invention are methods to detect cell-specific proteins, methods to detect cell-specific transcription factors or methods to detect physiological or morphological changes. All of these methods are per se well-known in the prior art. In particular, these methods use well-known protein or DNA or RNA detection processes and/or the reporter gene assays. Of course, immunoassays or assays using fluorescently or radioactively labelled proteins or nucleic acids including methods to measure enzymatic activities can be employed. Methods to detect morphological changes may include staining methods, visually based methods or the like.

Reprogrammed cells according to the present invention may be used for therapeutic, diagnostic or scientific purposes. In particular, these cells may be used in regenerative medicine and/or for replacement therapy.

The term "cell" not only refers to a single cell but also encompasses a cell line, a cell population or a cell clone.

The term "stem cells" refers to cells which have retained the capacity to proliferate and differentiate into one or different cell types. Stem cells created in accordance with the present invention are preferably pluripotent stem cells, i.e. stem cells which have retained the capacity to differentiate into distinct cell lineages and cell types or multipotent stem cells, retaining a more restricted differentiation potential.

It is understood that the term "stem cells" in accordance with the invention does not comprise human embryos. Furthermore, it is understood that the term "stem cells" does not comprise pluripotent stem cells which have been directly derived from a human embryo.

A "stem cell", as used herein, refers to any self-renewing pluripotent cell or multipotent cell or progenitor cell or precursor cell that is capable of differentiating into one or multiple cell types. Stem cells are thus cells able to differentiate into one or more than one cell type and have preferably an unlimited growth potential. Stem cells include those that are capable of differentiating into cells of osteoblast lineage, a mesenchymal cell lineage (e.g. bone, cartilage, adipose, muscle, stroma, including hematopoietic supportive stroma, and tendon). "Differentiate" or "differentiation", as used herein, refers to the process by which precursor or progenitor cells (i.e., stem cells) differentiate into specific cell types, e.g., osteoblasts. Differentiated cells can be identified by their patterns of gene expression and cell surface protein expression. "Dedifferentiate" or "dedifferentiation", as used herein, refers to the process by which lineage-committed cells (e.g., myoblasts or osteoblasts) reverse their lineage commitment and become precursor or progenitor cells (i.e., multipotent or pluripotent stem cells). Dedifferentiated cells can for instance be identified by loss of patterns of gene expression and cell surface protein expression associated with the lineage committed cells.

A "lineage-committed cell" as used herein, refers to any cell that has or will differentiate into a particular cell type or related cell types. Lineage committed cells include, for example, osteoblasts, myoblasts, chondrocytes, and adipocytes.

Totipotent stem cells are able to create all cell types of the body, including placental cells. The earlier stage fetal stem cells are considered totipotent, as well as the fertilised egg. They also have the ability to replicate in unlimited numbers without losing their potential.

Pluripotent stem cells are able to create cells of all three germ layers, namely the ecto-, endo- and mesoderm. They also have the ability to replicate in unlimited numbers without losing their potential.

Multipotent stem cells are able to produce cells of one or more germ layers or several types of tissues. They often have an already limited self-renewal ability.

Stem cells are thus cells able to differentiate into one or more than one cell type and have preferably an unlimited growth potential.

Progenitor cells can differentiate into one or more cell types but have a limited growth potential.

Adult stem cells are stem cells derived from an adult organism and might be multipotent or pluripotent.

Embryonic stem cells are derived from the inner mass of a blastula and are pluripotent. Embryonic stem cells are unique because they can develop into nearly all cell types, an attribute called pluripotency. But to access these cells, researchers must destroy a viable embryo. In this patent we are describing a way to create cells, with the characteristics of embryonic stem cells, without the need to destroy embryos or the use of embryonic stem cells.

Induced pluripotent stem cells "IPS" cells are pluripotent stem cells artificially derived from non-pluripotent cells, including somatic cells, adult multipotent stem cells or progenitor cells. In principal every cell containing a nuclei can be used as source for IPS cells.

Further preferred embodiments of the present invention are the subject matter of the subclaims.

The sequence listing shows the prior art DNA sequences encoding the mRNA nucleotide sequence molecules used in the present invention:

**Table 1**

| **SEQ ID No.** | **gene** | **accession-number** | **length** |
|---|---|---|---|
| **1** | **hNanog** | **NM_U24865** | **2098** |
| **2** | **mNanog** | **NM_028016** | **1356** |
| **3** | **rNanog** | **NM_001100781** | **2358** |
| **4** | **hSox2** | **NM_003106** | **2518** |
| **5** | **mSox2** | **NM_011443** | **2457** |
| **6** | **rSox2** | **NM_001109181** | **2323** |
| **7** | **hOct4** | **NM_002701** | **1411** |
| **8** | **mOct4** | **NM_013633** | **1346** |
| **9** | **hKIf4** | **NM_004235** | **2949** |
| **10** | **mKlf4** | **NM_010637** | **3057** |
| **11** | **rKlf4** | **NM_053713** | **2393** |
| **12** | **hTERT** | **NM_198253** | **4018** |
| **13** | **mTert** | **ENSMUSG00000021611** | **4237** |
| **14** | **rTERT** | **NM_053423** | **3378** |
| **15** | **hRonin** | **NM_020457** | **1903** |
| **16** | **mRonin** | **NM_021513** | **1832** |
| **abbreviations: h = human; m = mouse; r = rat** | | | |

The designations marked "NM" and ENSMUSG refer to the NM(NCBI)- and ENSMUSG-accession numbers as given under the publically available website http://www.ncib.nlm.nih.gov and http://www.ensembl.org.

SEQ ID No. 17 and 18 show the DNA sequence of primers used for cloning the human Nanog gene.

SEQ ID No. 19 and 20 show the DNA sequence of primers used for cloning the human Klf4 gene.

SEQ ID No. 21 and 22 show the DNA sequence of primers used for cloning the human Sox2 gene.

SEQ ID No. 23 and 24 show the DNA sequence of primers used for cloning the human Oct4 gene.

SEQ ID No. 25 and 26 show the DNA sequence of primers used for cloning the human Tert gene.

SEQ ID No. 27 and 28 show the DNA sequence of primers used for cloning the GFP gene.

The present invention will now be illustrated in more detail by way of an example and figures.

The figures show:

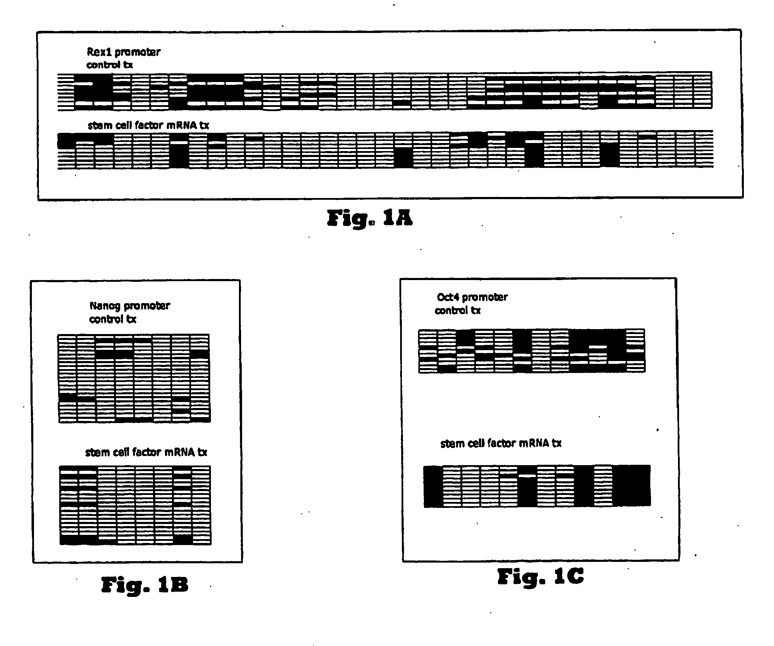

Figure 1: Promotor methylation of rex1, nanog and oct4 in human fibroblasts before and after reprogramming (Figure 1A: Rex; Figure 1AB: Nanog; Figure 1C: Oct4). CpG islands are represented as squares. Black squares represent methylated and white squares represent unmethylated CpG islands. All samples are primary fibroblasts from a young male donor.

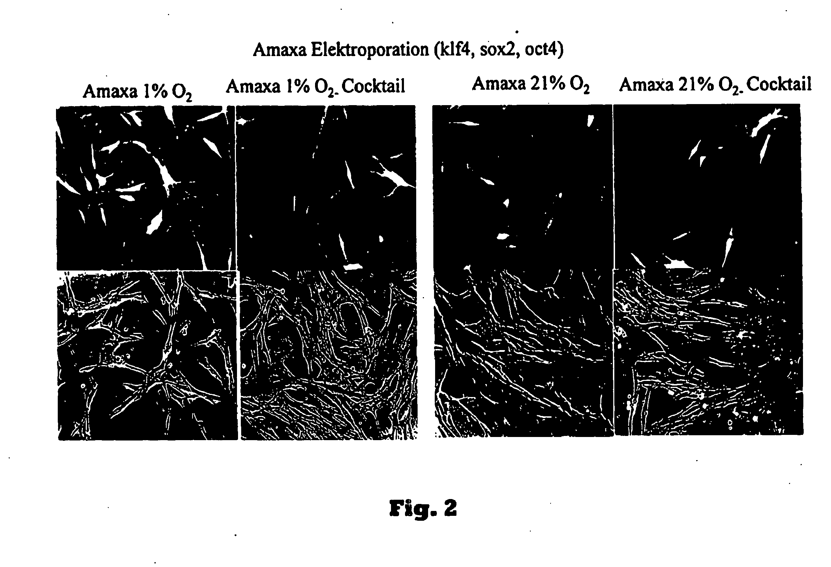

Figure 2: Fluorescence and morphology after amaxa electroporation (klf4, sox2, oct4): Human primary fibroblasts were transfected with 0,6 µg/µl mRNA per factor and 0,2 µg/µl GFP mRNA to visualize the transfection efficiency. Top line shows fluorescence pictures and the bottom line shows the morphology of the cells after 2 days.

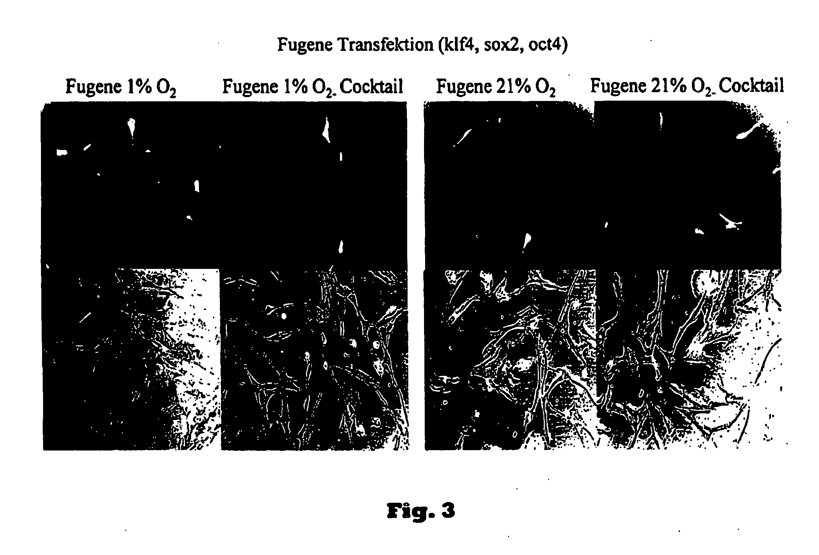

Figure 3: Fluorescence and morphology after Fugene transfection (klf4, sox2, oct4): Human primary fibroblasts were transfected with 0,6 µg/µl mRNA per factor and 0,2 µg/µl GFP mRNA to visualize the transfection efficiency. Top line shows fluorescence pictures and the bottom line shows the morphology of the cells after 7 days.

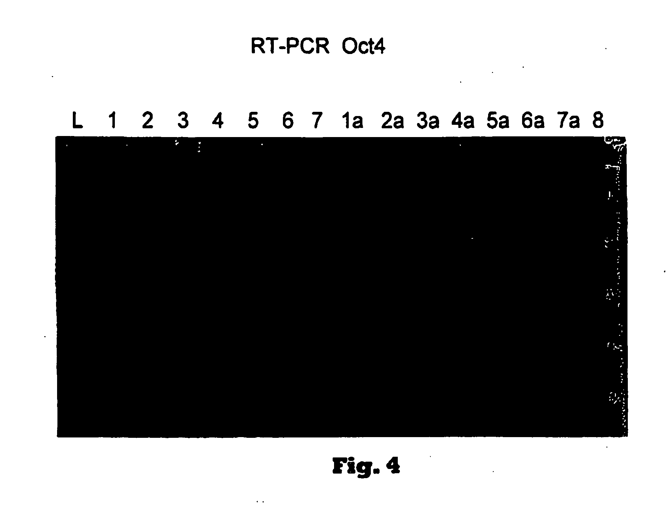

Figure 4: RT-PCR for Oct4: L: 20 bp Ladder (Fermentas); 1 hOct4 hypoxy factors 1x transfected; 2: hOct4 hypoxy factors 6x transfected; 3: hOct4 hypoxy factors 6x transfected + Cock; 4: hOct4 normoxy factors 1x transfected; 5: hOct4 normoxy factors 6x transfected; 6: hOct4 normoxy factors 6x transfected + Cock; 7: hOct4 normoxy factors 6x transfected + Cock + MG-132; 1a: hOct4 RNA after DNase digestion; 2a: hOct4 RNA after DNase digestion; 3a: hOct4 RNA after DNase digestion; 4a: hOct4 RNA after DNase digestion; 5a: hOct4 RNA after DNase digestion; 6a: hOct4 RNA after DNase digestion; 7a: hOct4 RNA after DNase digestion; 8: Oct4 negative control.

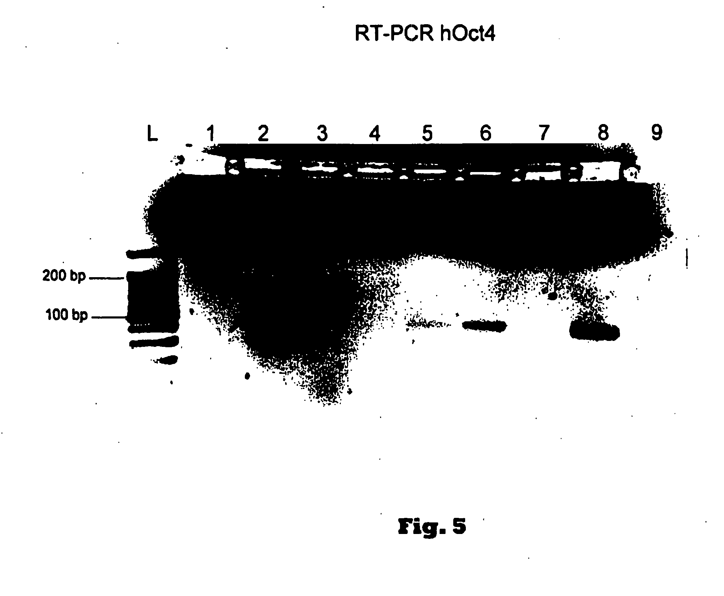

Figure 5: RT-PCR for hOct4: L: 20 bp Ladder (Fermentas); 1: hOct4 hypoxy factors 1x transfected (human fibroblasts); 2: hOct4 hypoxy factors 6x transfected (human fibroblasts transfected with mRNA); 3: hOct4 hypoxy factors 6x transfected + Cocktail (human fibroblasts transfected with mRNA and chemical reprogramming cocktail); 4: hOct normoxy factors 1x transfected; 5: hOct4 normoxy factors 6x transfected; 6: hOct4 normoxy factors 6x transfected + cocktail, 7: hOct4 normoxy 6x transfected factors + cocktail + MG-132; 8: hOct4 positive control (plasmid with hKlf4), 9: hOct4 negative control (water).

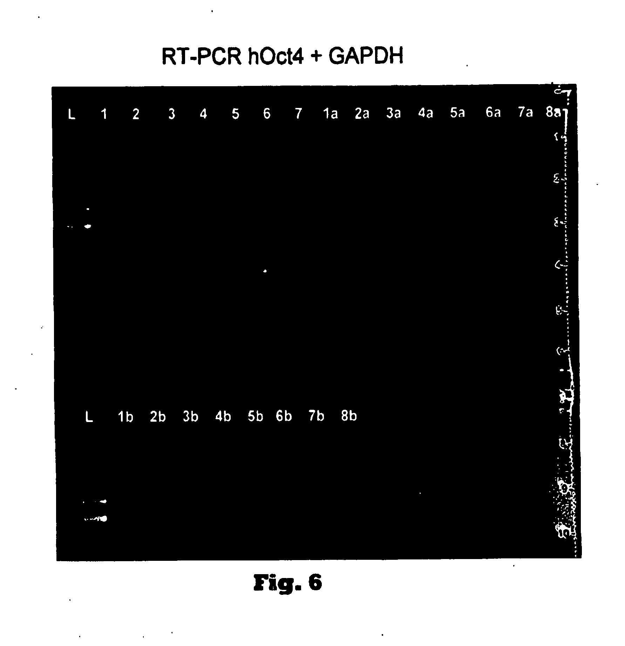

Figure 6: RT-PCR for hOct4 and GAPDH: 1: hOct4 hypoxy factors 1x transfected; 2: hOct4 hypoxy factors 6x transfected; 3: hOct4 hypoxy factors 6x transfected + chemical cocktail; 4: hOct4 normoxy factors 1x transfected; 5: hOct4 normoxy factors 6x transfected; 6 hOct4 normoxy factors 6x transfected + chemical cocktail; 7: hOct4 normoxy factors 6x transfected + chemical cocktail + MG-132; 1a hOct4 RNA after DNase digestion; 2a: hOct4 RNA after DNase digestion; 3a: hOct4 RNA after DNase digestion; 4a: hOct4 RNA after DNase digestion; 5a: hOct4 RNA after DNase digestion; 6a: hOct4 RNA after DNase digestion; 7a: hOct4 RNA after DNase digestion; 8a: Oct4 negative control; 1b: GAPDH hypoxy factors 1x transfected; 2b: GAPDH hypoxy factors 6x transfected; 3b GAPDH hypoxy factors 6x transfected + chemical cocktail; 4b: GAPDH normoxy factors 1x transfected; 5b: GAPDHnormoxy factors 6x transfected; 6b: GAPDH normoxy factors 6x transfected + chemical cocktail; 7b GAPDH normoxy factors 6x transfected + chemical cocktail + MG-132; 8b: GAPDH negative control.

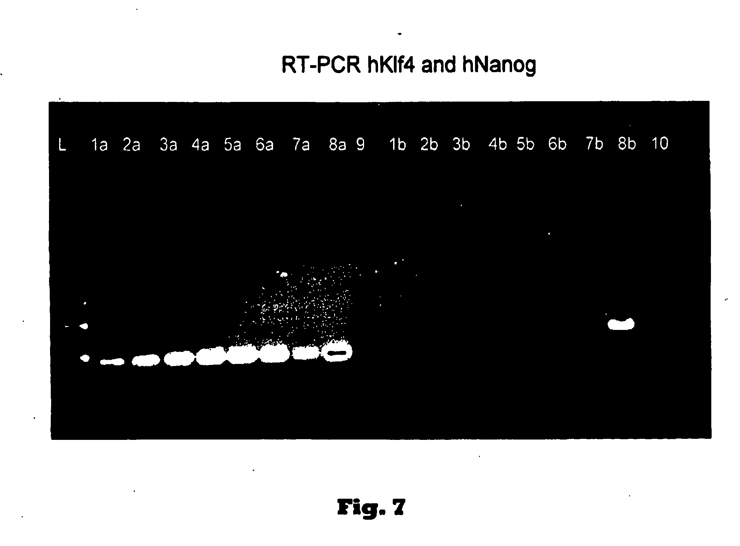

Figure 7: RT-PCR for hKlf4 and hNanog: L: 20 bp Ladder (Fermentas); 1a: hKlf4 hypoxy factors1x transfected (human fibroblasts); 2a: hKlf4 hypoxy factors 6x transfected (human fibroblasts with transfected mRNA); 3a: hKlf4 hypoxy factors 6x transfected + cocktail (human fibroblasts transfected with mRNA + chemical reprogramming cocktail); 4a: hKlf4 normoxy factors 1x transfected; 5a: hKlf4 normoxy factors 6x transfected; 6a: hKlf4 normoxy factors 6x transfected + cocktail, 7a: hKlf4 normoxy 6x transfected factors + cocktail + MG-132; 8a: hKlf4 pos. control (plasmid with hKlf4), 9a: hKlf4 neg. control (water) 1b: hNanog hypoxy factors 1x transfected (human fibroblasts); 2b: hNanog hypoxy factors 6x transfected (humane fibroblasts mit mRNA transfected); 3b: hNanog hypoxy factors 6x transfected + cocktail (human fibroblasts transfected with mRNA + chemical reprogramming cocktail); 4b: hNanog normoxy factors 1x transfected; 5b: hNanog normoxy factors 6x transfected; 6b: hNanog normoxy factors 6x transfected + cocktail, 7b: hNanog normoxy 6x transfected factors + cocktail + MG-132; 8b hNanog pos. control (plasmid with hNanog), 10: hNanog neg. control (water).

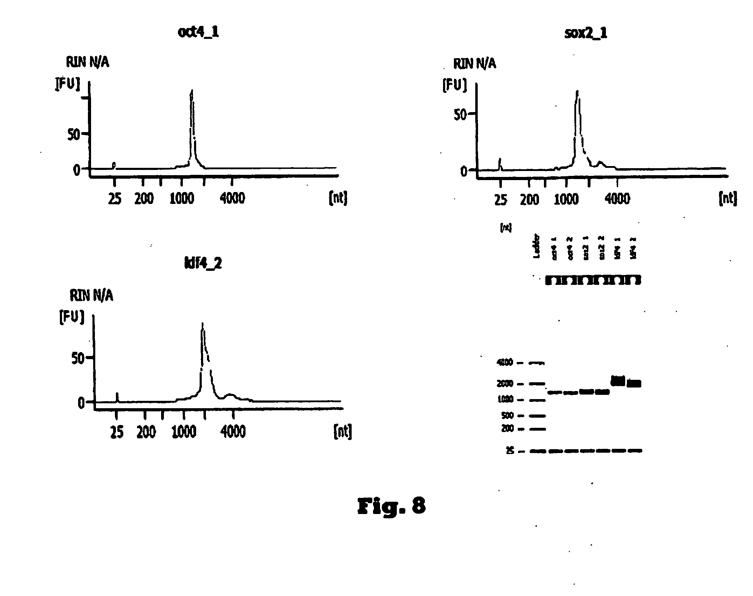

Figure 8: Results from RNA BioAnalyzer Analysis (Agilent Nano Chip; Agilent Technologies) for oct4, sox2, and klf4.

### Example

### 1. Preparation of reprogramming mRNA

### 1. A) Preparation of mRNA expression plasmids

For the construction of plasmids comprising the target genes in a vector with the T7-promotor for in *vitro* transcription, using PCR, digest sites before the start-codon and after the stop-codon were designed for direct cloning of the gene inserts (see Table 1) in the right direction into the pCR®II-Vector (Invitrogen).

The primers used are listed in Table 2.

**Table 2**

| Name gen | New digest sites for restriction enzymes | Primer sequence 5'-3' | Product size |
|---|---|---|---|
| Nanog_human | XbaI & NotI | SEQ ID No. 17 | 918 bp |
| Nanog_human | SpeI & Clal | SEQ ID No. 18 | |
| Klf4_human | XbaI & Notl | SEQ ID No. 19 | 637 bp |
| Klf4_human | HindIII & Clal | SEQ ID No. 20 | |
| Sox2_human | XbaI & BamHI | SEQ ID No. 21 | 996 bp |
| Sox2_human | HindIII & Clal | SEQ ID No. 22 | |
| Oct4_human | XbaI & Notl | SEQ ID No. 23 | 1100 bp |
| Oct4_human | BamHI & Clal | SEQ ID No. 24 | |
| Tert_human | XbaI & Notl | SEQ ID No. 25 | 783 bp |
| Tert_human | HindIII & Clal | SEQ ID No. 26 | |
| GFP | XbaI & Notl | SEQ ID No. 27 | 724 bp |
| GFP | HindIII & Clal | SEQ ID No. 28 | |

A PCR for obtaining the desired digest sites was carried out with the Platinum Taq-Polymerase (Invitrogen).

### Preparation of PCR-reaction on ice:

| | |
|---|---|
| 5 µl | 10x PCR Buffer |
| 1 µl | 10 mM dNTP mixture 0.2 mM each |
| 1.5 µl | 50 mM MgCl2 |
| 1 µl | Primer mix (10 µM each) 0.2 µM each |
| 1 µl | Template DNA |
| 0.2 µl | Platinum^{®} Taq DNA Polymerase 1.0 unit |
| to 50 µl | DEPC-H₂O |

### PCR-Program Biometra Tprofessional):

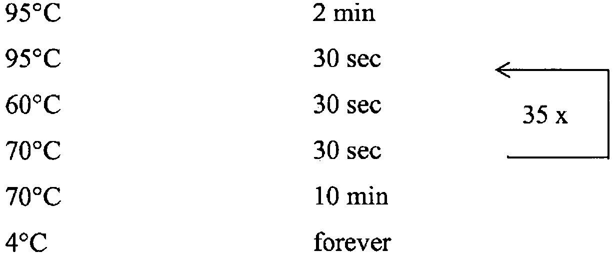

After electrophoresis in a 1 % TAE-agarose-gel, specific DNA-bands (for their size see Table 2) were cut out and the DNA extracted and purified with the QIAquick Gel Extraction Kit (from Qiagen)

### Procedure:

- excise the DNA fragment from the agarose gel with a scalpel
- weigh the gel slice
- add 3 volumes of Buffer QG to 1 volume of gel (for example, add 300 µl of Buffer QG to each 100 mg of gel)
- incubate at 50°C for 10 min (or until the gel slice has completely dissolved)
- add 1 gel volume of isopropanol to the sample and mix
- place a QlAquick spin column in a provided 2 ml collection tube
- to bind DNA, apply the sample to the QIAquick column, and centrifuge for 1 min at 13,000 rpm
- discard flow-through
- add 0.5 ml of Buffer QG to QlAquick column and centrifuge for 1 min at 13,000 rpm
- to wash, add 0.75 ml of Buffer PE to QlAquick column and centrifuge for 1 min at 13,000 rpm
- discard the flow-through
- centrifuge the QlAquick column for an additional 1 min at 13,000 rpm
- place QIAquick column into a 1.5 ml micro centrifuge tube
- to elute DNA, add 50 µl of H₂0 to the centre of the QIAquick membrane, let the column stand for 1 min, and then centrifuge for 1 min at 13,000 rpm

Digest of pCRII-Vector with the specific restriction enzymes for cloning of target-DNA

### Strategy for digest of vector pCRII:

vector pCRII digest with Xbal and Spel for target gene human Nanog
vector pCRII digest with Xbal and HindIII for target gene human Klf4
vector pCRII digest with Xbal and BamHI for target gene human Sox2
vector pCRII digest with Xbal and BamHI for target gene human Oct4
vector pCRII digest with Xbal and HindIII for target gene human TERT
vector pCRII digest with Xbal and HindIII for target gene GFP

### Preparation of digest on ice:

- 5 µl 10x Buffer
- ≥2 µg plasmid
- 2 µl restriction enzyme
- to 50 µl DEPC-water
- incubation for 1 h at 37°C (thermo block; Eppendorf)
- heat inactivation for 20 min by the specific temperature of the enzyme

### Ligation of the target-gene inserts DNA with the linear vector DNA with the T4-DNA-Ligase (Fermentas)

### Preparation of the ligation on ice:

- 50-400 ng vector DNA
- 50-400 ng insert DNA
- 2 µl 10x Buffer
- 0.2 µl (1u) T4-DNA Ligase
- to 20 µl DEPC-water
- vortex the tube and spin down in a micro centrifuge for 3-5 seconds
- incubate on room temperature for 1 h
- heat inactivation for 20 min

### 1. B) Transformation of the plasmids with chemical competent DH5α-bacteria

- add 1 µl of the plasmids to 10 µl chemical competent DH5α-bacteria
- incubation for 30 min on ice
- heat shock at 42°C for 45 sec
- add 250 µl SOC-Medium to the bacteria
- incubation at 37°C for 1h with shaking
- plate out the bacteria solution on LB-kanamycin-agarose-plate and incubate the plate for 12 hours in 37°C incubator
- pick one colony of the bacteria and put the colony in 5 ml LB-medium and incubate the solution for 12 hours in a bacteria incubator with shaking

### 1. C) Plasmid DNA purification with the NucleoSpin® Plasmid -KIT (Machery&Nagel)

- 5 ml of a saturated E. coli LB culture, pellet cells in a standard bench top micro centrifuge for 30 sec at 11,000 x g.
- discard the supernatant
- for cell lysis add 250 µl Buffer A1. Re-suspend the cell pellet completely by pipetting up and down
- add 250 µl Buffer A2
- mix gently by inverting the tube 10 times and incubate at room temperature for up to 5 min
- add 300 µl Buffer A3. Mix thoroughly by inverting the tube 10 times
- centrifuge for 5 min at 11,000 x g at room temperature
- place a NucleoSpin® Plasmid Column in a collection tube (2 ml) and load a 750 µl of the supernatant onto the column.
- centrifuge for 1 min at 11,000 x g.
- discard flow-through and place the NucleoSpin® Plasmid Column back into the collection tube (2 ml)
- for wash the membrane with DNA: Add 600 µl Buffer A4 (supplemented with ethanol).
- centrifuge for 1 min at 11,000 x g.
- discard flow-through and place the NucleoSpin® Plasmid Column back into the collection tube (2 ml)
- dry silica membrane: centrifuge for 2 min at 11,000x g and discard the collection tube (2 ml)
- elute DNA: place the NucleoSpin® Plasmid Column in a 1.5 ml micro centrifuge tube and add 50 µl H2O and incubate for 1 min at room temperature.
- centrifuge for 1 min at 11,000x g
- measure the DNA-concentration with the nanodrop
- DNA store at -20°C

### 1. D) Linearisation of the pCRII-hoct4, pCRII-hSox2, pCRII-hKlf4 plasmids for the in vitro transcription

- pCRII-hoct4 digest with BamHI
- pCRII-hSox2 digest with HindIII
- pCRII-hKlf4 digest with HindIII

### Preparation of digest on ice:

- 5 µl 10x Buffer
- 2 µg Plasmid
- 2 µl Restriction-Enzyme
- to 50 µl DEPC-H₂0
- incubation for 1 h at 37°C (thermo block; Eppendorf)
- heat inactivation for 20 min by the specific temperature of the enzyme
- heat inactivate both enzyme for 20 min at 80°C

### 1. E) Purification of the linearized pCRII-hoct4, pCRII-hSox2, pCRII-hKlf4 plasmids with QIAquick PCR Purification Kit (from Qiagen)

- add 5 volumes of Buffer PB to 1 volume of the digest sample and mix
- place a QIAquick spin column in a provided 2 ml collection tube
- to bind DNA, apply the sample to the QIAquick column and centrifuge for 60 s
- discard flow-through
- place the QIAquick column back into the same tube
- to wash, add 0.75 ml Buffer PE to the QIAquick column and centrifuge for 60 s
- discard flow-through and place the QIAquick column back in the same tube
- to dry the membrane, centrifuge the column for an additional 1 min
- place QIAquick column in a 1.5 ml micro centrifuge tube
- to elute DNA, add 10 µl H20 to the centre of the QIAquick membrane, let the column stand for 1 min, and centrifuge the column for 1 min
- measure the DNA-concentration with the nanodrop
- store DNA at -20°C

### 1. F) in vitro transcription of the linearized pCRII-hoct4, pCRII-hSox2, pCRII-hKlf4 plasmids with mMessage mMachine high yield capped RNA Transcription Kit (from Ambion) and add a poly(A) tail to RNA transcripts (Poly(A)-Tailing Kit from Ambion)

### Preparation of the in vitro transcription (mMESSAGE mMACHINE reaction):

- to 20 µl nuclease-free water
- 10 µl 2x NTP/CAP
- 2 µl 10x reaction buffer
- 1 µg linear template plasmid
- 2 µl enzyme mix
- pipette the mixture up and down gently
- short centrifugation to collect the reaction mixture at the bottom of the tube
- incubation for 2 h at 37°C
- add 1 µl TURBO DNase to the mRNA and mix well
- incubate at 37°C for 15 min
- directly after the DNase-treated reaction add the poly(A)-tail to the mRNA

### Preparation for poly(A)-tail reaction:

- 20µl mMESSAGE mMACHINE reaction
- 36µl nuclease-free water
- 20 µl 5x E-PAP Buffer
- 10 µl 25 mM MnCI2
- 10 µl mM ATP
- 4µl E-PAP
- mix gently
- incubate at 37°C for 1 h
- place reaction on ice
- lithium chloride precipitation of the mRNA with poly(A)-tail:
- add 30 µl LiCl precipitation solution
- mix thoroughly
- chill for 30 min at -20°C
- centrifuge at 4°C for 15 min at maximum speed to pellet the RNA
- remove the supernatant
- wash the pellet once with 1 ml 70% ethanol
- centrifuge at 4°C for 15 min at maximum speed to pellet the RNA
- remove the 70% ethanol
- air-dry the pellet
- solve the RNA-Pellet with 30-50 µl nuclease-free-water
- the concentration of the RNA is measured with the nanodrop
- the quality of the RNA is measured with the Agilent nano-chip

### 2. Preparation of target cells

### 2. A) Preparation of mouse and rat MSCs

- kill the rat or mouse with CO2-gassing
- spray the rats and mice with 70% ethanol to kill bacteria and fungal spores
- remove the coat from the skin
- remove the back legs cleanly at the hip joint
- under sterile conditions, remove all soft tissue and separate the bones
- remove the growth plates both femur and tibia
- insert holes with a needle into the bones on both ends
- place the bones in eppendorf tubes (for mice) or in falcon tubes (for rats) and spin at 2000 rpm for 1 min (all of the bone marrow will be deposited in the bottom of the tube sitting in spacers); remove the bones and spacer
- re-suspend the marrow in 0,5 ml medium (DMEM low glucose; 10 % FCS; 1 % Pen/Strep)
- disseminate the marrow of one leg from a rat in 1 T-75 flask with 12 ml medium
- disseminate the marrow of two legs from a mouse in 1 10 cm petri-dish with 12 ml medium

### 2. B) Preparation of human MSC

- get a bone marrow aspirate
- re-suspend in DMEM (low glucose, 10%FCS)
- plate out in a tissue culture flask
- growth for 5 days and then change media
- thereafter change the medium twice weekly
- culture in tissue culture flask until 80% confluency and subculture until needed

### 2. C) Preparation of mouse and rat fibroblasts

- cut off the tail
- shave the tail with a scalpel
- spray the tail with 70% ethanol to kill bacteria and fungal spores
- remove the skin from the tail
- place the skin in a 10 cm petri dish
- under sterile conditions, cut the skin in small pieces (5 x 5 mm)
- digest the skin-pieces with 0,05 % Trypsin EDTA for 20 min in 37°C CO₂-incubator
- wash the trypsin from the skin-dishes with medium (DMEM high glucose, 10 % FCS, 1 % Pen/Strep), remove the medium and wash again for 3-4 times
- then incubate the skin pieces in 37°C CO₂-incubator and change the medium every day, after 1 week the fibroblasts grow out

### 2. D) Preparation of human fibroblasts

- place prepuce in petri dish
- wash two times with PBS
- remove fat
- wash with PBS one time
- cut the skin in small pieces (2 mm)
- add 10 ml dispase (2 U/ml)
- incubate at 4°C for 16-18 h
- discard dispase solution
- add 5 ml PBS
- separate dermis and epidermis
- transfer dermis to new dish and add 10 ml collagenase (500 U/ml)
- make very small pieces
- transfer pieces into a 50 ml falcon tube with solution and incubate for 45 min (37°C; 5% C0₂)
- centrifuge at 1000 rpm for 5 min
- discard supernatant and resuspend pellet in DMEM (10% FCS)
- centrifuge again
- resuspend pellet in 2 ml DMEM (10% FCS) and transfer into a T75 cell culture flacon
- expanded cells until use

### 3. mRNA Transfection

### 3. A) mRNA-transfection of human fibroblasts, human MSC, rat fibroblasts, rat MSC, mouse fibroblasts and mouse MSC with AMAXA, FugeneHD, Lipofectamine™ LTX Reagent and PLUS™ Reagent

### 1) AMAXA-Transfection with the Human Dermal Fibroblast Nucleofector® Kit (6 well-plate):

### Preparation for one electroporation in certified Amaxa cuvette:

- mRNA of oct4, sox2 and klf4 in sum of 2 µg
- 4 x 10⁵ cells
- 100 µl Nucleofector® Solution
- choose the program U-023 by the Amaxa-electroporation-machine
- re-suspend the cells in the cuvette with 500 µl fibroblast or MSC-Medium
- provide 5 ml fibroblast or MSC-Medium with or without Cocktail in every well of a 6 well-plate
- plate out 100.000 cells in each well of a 6 well-plate

### Cocktail composition:

- Resveratrol 0,05 mM
- Selenium 0,1 µM
- EGCG ((-)-Epigallocatechin gallate) 0,01 µM
- TSA (Trichostatin A) 0,015 µM
- Reversine 1 µM
- VPA (2-Propylpentanoic acid free acid) 6 µM
- 5'Aza (5-Aza-2'-deoxycytidine) 1 µM
- at day 1: the cocktail is with 5'Aza and without TSA; after 48 hours 5'Aza replaced by TSA
- every 72 hours new transfection with FuGENE® HD Transfection Reagent (Roche)

### Transfection FuGENE® HD Transfection Reagent (Roche)

- before transfection: change to antibiotic free medium

### Transfection procedure

- Dilute mRNA with serum-free and antibiotic-free medium to a concentration of 2 µg mRNA at a volume of 100 µl for each well (6 well-plate)
- Pipet the 8 µl FuGENE® HD Transfection Reagent (Ratio: 8:2 Transfection reagent to mRNA) directly into the medium pipette up and down
- Incubate the transfection reagent: mRNA complex for 15 minutes at room temperature
- Add the transfection complex to the cells in a drop-wise manner
- Swirl the wells to ensure distribution over the entire plate surface
- after 4-6 hours change to medium (contains 10 % FCS, 1 % Pen/Strep) with or without cocktail
- one transfected 6 well plate incubate at 21 % O2-condition (normoxy) and a second plate incubate at 1 % O2-condition (hypoxy) at 37°C incubator

### 3) Alternative transfection with Lipofectamine™ LTX Reagent or PLUS™ Reagent (Invitrogen)

### a) Transfection FuGENE® HD Transfection Reagent (Roche)

- plate out 7000 cells in 500 µl medium per well of a 24 well-plate
- incubate over night
- for transfection: change to antibiotic free medium
- dilute 0,5 µg mRNA in 25 µl serum-free and antibiotic-free medium for each well (24 well-plate)
- pipette the FuGENE® HD Transfection Reagent 2 µl (Ratio: 8:2 Transfection reagent to mRNA) directly into the medium
- pipette up and down
- incubate the transfection reagent: mRNA complex for 15 minutes at room temperature
- add the transfection complex to the cells in a drop-wise manner
- swirl the wells to ensure distribution over the entire plate surface
- after 4-6 hours change to medium (contains 10 % FCS, 1 % Pen/Strep) with or without cocktail

### b) Lipofectamine™ LTX Reagent and PLUS™ Reagent (Invitrogen)

- plate out 7000 cells in 500 µl medium per well of a 24 well-plate
- incubation over night
- before transfection: change to antibiotic free medium
- dilute 0,5 µg mRNA in 100 µl serum-free and antibiotic-free medium for each well (24 well-plate)
- mix gently
- mix PLUS™ Reagent gently before use
- add 0,5 µl PLUS™ Reagent in the mRNA-Medium mix, mix gently and incubate 5 min at room temperature mix Lipofectamine™ LTX Reagent gently before use
- add 1,25 µl directly to the diluted mRNA; mix gently
- incubate for 30 min at room temperature
- add the 100 µl mRNA- Lipofectamine™ LTX Reagent complex to the well
- mix gently by rocking the plate back and forth
- incubate the cells at 37°C in the CO2 incubator for 4-6 hours
- change to medium with or without cocktail
- during the optimization of the transfection efficiency → in crease of the amount of mRNA concentration at 1 µg per well (24 well plate)
- every 72 h repeat the transfection with FuGENE^{®} HD or Lipofectamine™ LTX Reagent and PLUS™ Reagent

### 4. Protease inhibitor treatment (MG132) to increase the half-life of the proteins produced by the transfected mRNA:

- 48h after transfection: treatment of two wells in every plate with MG-132 (10 µM/well) with and without cocktail for 6 h, then change medium

### 5. Results

Analysis of mRNA amount (per PCR) of the transfected or internal genes (oct4, nanog, hTERT, sox2, klf4) and promoter analysis (of nanog, sox, oct4)

The reprogramming results are set forth in figures 1 to 8.

### 6. Tools and Methods

### 6.1 RNA-Isolation with TriFaSt™(Peglab)

- 1 ml Trifast per 10 cm2 of the culture dish area
- samples can be stored for a long time at -80°C or can be directly used
- samples should be kept for 5 minutes at room temperature
- addition of 0.2 ml of chloroform
- shake samples by hand vigorously for 15 seconds
- incubation for 3 minutes at room temperature
- centrifugation at 12.000x g
- transfer the aqueous phase with RNA to new tube (save the interphase and organic phase at 4°C for the isolation of DNA and proteins).
- precipitation of the RNA with 0.5 ml of isopropanol per 1 ml of Tri-Fast™ used for the initial homogenization.
- incubation on ice for 15 min
- centrifuge for 10 minutes at 4°C at 12.000x g
- remove the supernatant
- wash the RNA pellet twice with 75% ethanol by vortexing
- subsequent centrifugation for 8 minutes at 7,500x g (4°C)
- remove the excess isopropanol from the RNA pellet by airdrying
- re-suspend the RNA pellet in RNase-free water (DEPC-H20) → 30 µl - 50 µl
- dissolve the RNA pellet by passing the solution through a pipette tip several times
- incubating the solution for 10 min at 55°C
- pipetting it up and down.
- Heating the sample at 55-60°C might help to dissolve the pellet
- measure the RNA-Concentration → NanoDrop
- samples store at -80°C for 2-3 months

### 6.2 DNA-Isolation

- Remove any left aqueous phase after RNA removal
- precipitate the DNA with 0.3 ml of 100% ethanol per ml Tri-Fast™
- mix well by inversion
- store the samples at room temperature for 2-3- minutes
- centrifugation at 2,000x g at 4°C for 5 minutes
- move the DNA supernatant (store it at 4°C for the protein isolation)
- DNA pellet washed twice with 1 ml 0.1 M sodium citrate in 10% ethanol
- At each wash step keep the DNA in 0.1 M sodium citrate /10% ethanol for 30 minutes at room temperature (with periodic mixing)
- centrifuge at 4°C for 5 minutes at 2,000x g
- after these two washes suspend the DNA pellet in 2 ml of 75% ethanol
- keep it at room temperature with periodic mixing for 15 minutes
- centrifuge at 2,000 x g for 5 minutes at 4°C
- dry the DNA pellet briefly for 5-10 minutes under vacuum and
- dissolve it in 8 mM NaOH by slowly passing the pellet through a wide bore pipet.
- adjust the final concentration of DNA to 0.2-0.3 µg/µl with 8 mM NaOH

### 6.3 RNA treatment with DNase I (Fermentas):

- 1 µg RNA
- 1 µl 10x reaction buffer with MgCI2
- 1 µl (1 u) DNase
- to 9 µl DEPC-H2O
- incubate at 37°C for 30 minutes
- inactivation.of DNase 1: Add 1 µl 25mM EDTA and incubate at 65°C for 10 min

### 6.4 RT-PCR with SuperScript™ III Reverse Transcriptase (Invitrogen)

### Reaction condition:

- 1 µl oligo(dT)20 Primer
- 1 µg RNA
- 1µl 10 mM dNTP Mix (10 mM each dATP, dGTP, dCTP and dTTP)
- to 13 µl DEPC-H20
- heat mixture to 65°C for 5 minutes
- incubate on ice for 1 min

### Add:

- 4 µl 5x First-Strand Buffer
- 1 µl 0.1 M DTT
- 1 µl SuperScript™ III RT (200 units/µl)
- mix by pipetting gently up and down
- incubate at 50°C for 60 min
- inactivate the reaction by heating at 70°C for 15 minutes
- store the cDNA at -20°C

### 6.5 PCR of human oct4 (control the human fibroblasts):

Primersequence: hoct4_S: gaggatcaccctgggatataca
   hoct4_as: agatggtcgtttggctgaatac
Product size: 100 bp

### 6.6 PCR with Patinum Taq-Poymerase (Invitrogen)

Preparation of PCR-reaction on ice:
- 5 µl 10x PCR Buffer
- 1 µl 10 mM dNTP mixture 0.2 mM each
- 1.5 µl 50 mM MgCI2
- 1 µl Primer mix (10 µM each) 0.2 µM each
- 1 µl Template DNA
- 0,2 µl Platinum® Taq DNA Polymerase 1.0 unit
- to 50 µl DEPC-H₂0

### PCR-Program (Biometra Tprofessional):

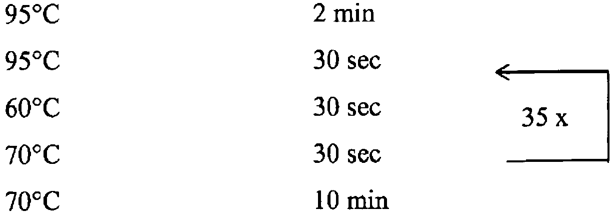

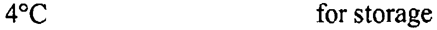

PCR-control with 3% TAE-agarose-gei for electrophoresis

### 6.7 Protocol to produce iPS cells

### 6.7.1 BD Matrigel™ hESC-qualified Matrix

- put the sterilized tip box and eppendorf tubes over night in - 20°C freezer
- thaw the Matrigel bottle on ice in the 4°C fridge overnight
- aliquot Matrigel for hESC upon arrival, store at -80°C, only thaw once and do not re-freeze to avoid breakdown of growth factors
- for coating purposes, dilute Matrigel appropriately by using serum-free medium (0.3 µg/µl for iPS, 1 µg/µl for hESC)
- add 50 µl Matrigel-dilution per cm2 growth surface
- incubate 60 min at RT under sterile conditions or overnight at 4°C
- soak off remaining Matrigel/medium
- Medium: mTeSR1 (Stem Cell Technology)

The coating recipe (0.3 µg/µl) is dependent on the cell type that is used to generate iPS. It is recommended to coat and not gel plastics for hESC culture using 1 ug Matrigel/µl. Use plates immediately or store for max 7 days at 4°C covered with serum-free medium under aseptical conditions (sealed).
The coated Matrigel plates should always be processed, stored and used exactly the same way to avoid variabilities (mainly through breakdown of growth factors). In the beginning or for establishment it is necessary to titrate a coating volume and -concentration (for instance 3 dilutions 0.3, 0.6, 1 ug/ul) that suit your specific type of cells best.

### 6.7.2 BD Matrigel™ Basement Membrane Matrix, Growth Factor Reduced (GFR)

- aliquote the matrigel such as hESC-qualified Matrigel
- thaw tube overnight on ice at 4°C
- dilute with 6 ml cold basal media and mix well
- add 1 ml per well of 6 well plate
- incubate the plate at room temperature for one hour or overnight at 4°C
- plate may either be used immediately or stored at 4°C (plate are be good for least one week)
- remove the excess liquid and wash once with basal medium
- basal medium component: D-MEM/F-12; 20% KO Serum Replacer; 1% Non-essential Amino Acids; 1mM L-Glutamine, 0.1 mM 2-Mercaptoethanol; 100 ng/ml bFGF
- use mouse cells then add LIF-supernatent to the basal medium

### 6.7.3 Production of LIF-supernatant

- coat petri dish (145 mm) with 0,1 % gelatine (Bovine Type B, Sigma)
- plate 3 x 10¹⁰ LIF producing feeder cells SNL in every dish
- Medium component: D-MEM high glucose, 10% FCS, 1% Pen/Strep; 1 % L-Glutamine
- after 24 hours collect the medium from the dish and filter through a 0,22 µm filter
- aliquote the medium containing LIF in 500 µl aliquots and store at -20°C
- change media after 24 hours or 48 hours

## Claims

1. A process for preparing reprogrammed mammalian cells in vitro without the use of a virus, comprising
a) providing isolated mammalian cells to be reprogrammed,
b) transfecting one or more reprogramming mRNA molecules into the cells provided in step a), wherein the reprogramming mRNA molecules are encoding at least one reprogramming protein selected from the group consisting of Ronin, Oct4, Klf4, Sox2, Nanog and TERT,
c) culturing the cells obtained in step b) in a cell culture medium and under a condition suitable to allow translation of the transfected reprogramming mRNA molecules, wherein the cell culture medium contains at least one transient proteolysis inhibitor, so as to obtain reprogrammed cells, wherein said reprogrammed cells are mammalian cells exhibiting a pluripotent or multipotent stem cell character.

2. The process according to claim 1, wherein the condition suitable to allow translation is an oxygen content in the cell culture medium from 0.5% to 21 %.

3. The process according to claim 1 or 2, wherein the condition suitable to allow translation is a temperature from 30°C to 38°C.

4. The process according to any one of the preceding claims, wherein the cell culture medium has a glucose content of 0.1 g/l to 4.6 g/l.

5. The process according to any one of the preceding claims, wherein the cell culture medium contains at least one inducing substance selected from the group consisting of reversine, resveratrol, selenium, selenium containing compounds, EGCG (epigallocatechin-3-gallate), valproic acid, salts of valproic acid and sodium valproate.

6. The process according to any of the preceding claims, wherein the transient proteolysis inhibitor is selected from the group consisting of protease inhibitor, proteasome inhibitor and lysosome inhibitor.

7. The process according to claim 6, wherein said proteasome inhibitor is selected from the group consisting of MG132, TMC-95A, TS-341, and MG262.

8. The process according to claim 6, wherein said protease inhibitor is selected from the group consisting of aprotinin, G-64 and leupeptine-hemisulfat.

9. The process according to claim 6, wherein said lysosome inhibitor is ammonium chloride.

10. The process according to any one of the preceding claims, wherein the cells to be reprogrammed are human cells.

11. A process for inducing the dedifferentiation of mammalian cells, wherein a process according to any one of claims 1 to 10 is carried out.

12. A process for improving the expansion of mammalian stem cells in vitro, wherein a process according to any one of claims 1 to 11 is carried out, comprising
a) providing isolated mammalian cells to be reprogrammed,
b) transfecting one or more reprogramming mRNA molecules into the cells provided in step a), wherein the reprogramming mRNA molecules are encoding at least one reprogramming protein selected from the group consisting of Ronin, Oct4, Klf4, Sox2, Nanog and TERT,
c) culturing the cells obtained in step b) in a cell culture medium and under a condition suitable to allow translation of the transfected reprogramming mRNA molecules, wherein the cell culture medium contains at least one transient proteolysis inhibitor, so as to obtain reprogrammed cells, wherein said reprogrammed cells are mammalian cells exhibiting a pluripotent or multipotent stem cell character,
wherein said culturing in step c) is **characterized by** prolonging expansion of the pluripotent or multipotent stem cells obtained, whereby said stem cells to be expanded are kept in a dedifferentiated status.

13. A process for the rejuvenation of aged mammalian cells, wherein a process according to any one of claims 1 to 11 is carried out

## Patentansprüche

1. Verfahren für die Präparation reprogrammierter Säugerzellen in vitro ohne die Verwendung von Viren, umfassend:
a) Bereitstellung isolierter Säugerzellen zur Reprogrammierung,
b) Transfizierung eines oder mehrerer reprogrammierender mRNA-Moleküle in die in Schritt a) bereitgestellten Zellen, wobei die reprogrammierenden mRNA-Moleküle mindestens ein reprogrammierendes Protein, ausgewählt aus der Gruppe bestehend aus Ronin, Oct4, Klf4, Sox2, Nanog und TERT, kodieren,
c) Kultivierung der in Schritt b) erhaltenen Zellen in einem Zellkulturmedium und unter Bedingungen, die für die Translation der transfizierten reprogrammierenden mRNA-Moleküle geeignet sind, wobei das Zellkulturmedium mindestens einen kurzlebigen Proteolyse-Inhibitor für die Gewinnung reprogrammierter Zellen umfasst, wobei die besagten reprogrammierten Zellen Säugerzellen sind, die Eigenschaften pluripotenter oder multipotenter Stammzellen aufweisen.

2. Verfahren nach Anspruch 1, wobei die für die Translation geeignete Bedingung ein Sauerstoffgehalt von 0,5 % bis 21 % in dem Zellkulturmedium ist.

3. Verfahren nach Anspruch 1 oder 2, wobei die für die Translation geeignete Bedingung eine Temperatur von 30° C bis 38° C ist.

4. Verfahren nach einem der vorangehenden Ansprüche, wobei das Zellkulturmedium einen Glukosegehalt von 0,1 g/l bis 4,6 g/l hat.

5. Verfahren nach einem der vorangehenden Ansprüche, wobei das Zellkulturmedium mindestens eine induzierende Substanz, ausgewählt aus der Gruppe bestehend aus Reversin, Resveratrol, Selen, Selen enthaltende Verwindungen, EGCG (Epigallecatechin-3-gallat), Valproinsäure, Salze der Valproinsäure und Natriumvalproat, enthält.

6. Verfahren nach einem der vorangehenden Ansprüche, wobei der kurzlebige Proteolyse-Inhibitor ausgewählt ist aus der Gruppe bestehend aus Protease-Inhibitor, Proteasom-Inhibitor und Lysosom-Inhibitor.

7. Verfahren nach Anspruch 6, wobei der Proteasom-Inhibitor ausgewählt ist aus der Gruppe bestehend aus MG132, TMC-95A, TS-341 und MG262.

8. Verfahren nach Anspruch 6, wobei der Protease-Inhibitor ausgewählt ist aus der Gruppe bestehend aus Aprotinin, G-64 und Leupeptin-Hemisulfat.

9. Verfahren nach Anspruch 6, wobei der Lysosom-Inhibitor Ammoniumchlorid ist.

10. Verfahren nach einem der vorangehenden Ansprüche, wobei die zu reprogrammierenden Zellen menschliche Zellen sind.

11. Verfahren für das Induzieren der Dedifferenzierung von Säugerzellen, wobei ein Verfahren nach einem der Ansprüche 1 bis 10 durchgeführt wird.

12. Verfahren für die Verbesserung der Expansion von Säugerstammzellen in vitro, wobei ein Verfahren nach einem der Ansprüche 1 bis 11 durchgeführt wird, umfassend:
a) Bereitstellung isolierter Säugerzellen zur Reprogrammierung,
b) Transfizierung eines oder mehrerer reprogrammierender mRNA-Moleküle in die in Schritt a) bereitgestellten Zellen, wobei die reprogrammierenden mRNA-Moleküle mindestens ein reprogrammierendes Protein, ausgewählt aus der Gruppe bestehend aus Ronin, Oct4, Klf4, Sox2, Nanog und TERT, kodieren,
c) Kultivierung der in Schritt b) erhaltenen Zellen in einem Zellkulturmedium und unter Bedingungen, die für die Translation der transfizierten reprogrammierenden mRNA-Moleküle geeignet sind, wobei das Zellkulturmedium mindestens einen kurzlebigen Proteolyse-Inhibitor für die Gewinnung reprogrammierter Zellen umfasst, wobei die besagten reprogrammierten Zellen Säugerzellen sind, die Eigenschaften pluripotenter oder multipotenter Stammzellen aufweisen,
wobei die besagte Kultivierung in Schritt c) **gekennzeichnet ist durch** das Verlängern der Expansion der gewonnenen pluripotenten oder multipotenten Stammzellen und wobei die zu expandierenden Stammzellen in einem dedifferenzierten Zustand gehalten werden.

13. Verfahren für die Verjüngung gealterter Säugerzellen, wobei ein Verfahren nach einem der Ansprüche 1 bis 11 durchgeführt wird.

## Revendications

1. Procédé de préparation in vitro de cellules de mammifères reprogrammées sans utiliser un virus, consistant à :
a) fournir des cellules de mammifères isolées à reprogrammer;
b) transfecter une ou plusieurs molécules d'ARNm de reprogrammation dans les cellules fournies à l'étape a), les molécules d'ARNm de reprogrammation codant au moins une protéine de programmation sélectionnée dans le groupe constitué de Ronin, d'Oct4, de Klf4, de Sox2, de Nanog et de TERT ;
c) cultiver les cellules obtenues à l'étape b) dans un milieu de culture cellulaire et dans des conditions adéquates pour permettre la translation des molécules d'ARNm de reprogrammation transfectées, le milieu de culture cellulaire contenant au moins un inhibiteur de protéolyse transitoire, de façon à obtenir des cellules reprogrammées, ces dernières étant des cellules de mammifères présentant un caractère de cellules souches pluripotentes ou multipotentes.

2. Procédé selon la revendication 1, la condition adéquate pour permettre la translation étant une teneur en oxygène dans le milieu de culture cellulaire allant de 0,5% à 21%.

3. Procédé selon la revendication 1 ou 2, la condition adéquate pour permettre la translation étant une température allant de 30°C à 38°C.

4. Procédé selon une quelconque des revendications précédentes, le milieu de culture cellulaire ayant une teneur en glucose allant de 0,1 g/l à 4,6 g/l.

5. Procédé selon une quelconque des revendications précédentes, le milieu de culture cellulaire contenant au moins une substance inductrice choisie dans le groupe constitué de réversine, de resvératrol, de sélénium, de sélénium contenant des composés, d'EGCG (épigallocatéchine-3-gallate), d'acide valproïque, de sels de l'acide valproïque, et de valproate de sodium.

6. Procédé selon une quelconque des revendications précédentes, l'inhibiteur de protéolyse transitoire étant choisi dans le groupe constitué de l'inhibiteur de protéase, de l'inhibiteur de protéasome et de l'inhibiteur de lysosome.

7. Procédé selon la revendication 6, ledit inhibiteur de protéasome étant choisi dans le groupe constitué de MG132, de TMC-95A, de TS-341 et de MG262.

8. Procédé selon la revendication 6, ledit inhibiteur de protéase étant choisi dans le groupe constitué d'aprotinine, de G-64 et d'hémisulfate de leupeptine.

9. Procédé selon la revendication 6, ledit inhibiteur de lysosome étant le chlorure d'ammonium.

10. Procédé selon une quelconque des revendications précédentes, les cellules à reprogrammer étant des cellules humaines.

11. Procédé pour induire la dédifférenciation de cellules de mammifères, selon lequel un procédé selon une quelconque des revendications 1 à 10 est réalisée.

12. Procédé pour améliorer l'expansion in vitro de cellules souches de mammifères, selon lequel un procédé selon une quelconque des revendications 1 à 11 est réalisée, ledit procédé consistant à :
a) fournir des cellules de mammifères isolées à reprogrammer ;
b) transfecter une ou plusieurs molécules d'ARNm de reprogrammation dans les cellules fournies à l'étape a), les molécules d'ARNm de reprogrammation codant au moins une protéine de programmation choisie dans le groupe constitué de Ronin, d'Oct4, de Klf4, de Sox2, de Nanog et de TERT;
c) cultiver les cellules obtenues à l'étape b) dans un milieu de culture cellulaire et dans des conditions adéquates pour permettre la translation des molécules d'ARNm de reprogrammation transfectées, le milieu de culture cellulaire contenant au moins un inhibiteur de protéolyse transitoire, de façon à obtenir des cellules reprogrammées, ces dernières étant des cellules de mammifères présentant un caractère de cellules souches pluripotentes ou multipotentes;
ladite culture à l'étape c) étant **caractérisée par** le prolongement de l'expansion des cellules souches pluripotentes ou multipotentes obtenues, lesdites cellules souches à étendre étant maintenues dans un état dédifférencié.

13. Procédé pour le rajeunissement de cellules de mammifères âgées, selon lequel un procédé selon une quelconque des revendications 1 à 11 est réalisé.
